(19) Europäisches Patentamt / European Patent Office / Office européen des brevets

(11) **EP 1 977 691 A1**

(12) **EUROPEAN PATENT APPLICATION**
published in accordance with Art. 158(3) EPC

(43) Date of publication:
**08.10.2008 Bulletin 2008/41**

(51) Int Cl.:
***A61B 5/05*** (2006.01)

(21) Application number: **07706587.8**

(22) Date of filing: **11.01.2007**

(86) International application number:
**PCT/JP2007/050240**

(87) International publication number:
**WO 2007/083563 (26.07.2007 Gazette 2007/30)**

(84) Designated Contracting States:
**DE ES FR GB IT**

(30) Priority: **18.01.2006 JP 2006010262**

(71) Applicant: Omron Healthcare Co., Ltd.
**Kyoto 615-0084 (JP)**

(72) Inventors:
• **SATO, Tetsuya**
**Kyoto-shi, Kyoto 615-0084 (JP)**

• **TOGOE, Yasuyuki**
**Kyoto-shi, Kyoto 615-0084 (JP)**

(74) Representative: **Wilhelms · Kilian & Partner**
**Patentanwälte**
**Eduard-Schmid-Strasse 2**
**81541 München (DE)**

(54) **BODY COMPOSITION METER ENABLING THE RECOGNITION OF BODY PARTS USED IN COMPUTING COMPONENTS OF THE COMPOSITION**

(57)  A body composition measuring instrument for measuring a body composition of a whole body of a subject includes a detecting section (11) for detecting a plurality of potential differences at each of a plurality of body sites including a whole body, both hands, and both feet by using hand electrodes and foot electrodes; first and second body composition calculating units (103, 105) for calculating the body composition of the whole body based on at least one of the potential differences detected by the detecting section (11) and body information of the subject; and an informing unit (108) for informing the information related to the body site to be detected of the potential difference used in the calculation of the body composition of the whole body.

Fig. 3

EP 1 977 691 A1

**Description**

TECHNICAL FIELD

The present invention relates to body composition measuring instruments, in particular, to a body composition measuring instrument capable of calculating a composition component (body composition) of a body through a bioelectric impedance method.

BACKGROUND ART

There has been a body composition measuring instrument for calculating the body composition of a subject through the bioelectric impedance method from the prior art. Such body composition measuring instrument is used for health management of the subject.

Japanese Laid-Open Patent Publication No. 2005-230120 (hereinafter referred to as patent document 1) has proposed a body composition measurement device including a means for determining the measurement state of each site based on the impedance of each measured site, and a mode switching means for switching the measurement mode of the impedance based on the measurement stage in the body composition measuring instrument for calculating the body composition of the subject by arranging a current application electrode and a voltage measurement electrode on both hands and both feet, and measuring the impedance of each site of the living body. The measurement mode suited to the subject can be then automatically selected, and body composition data having high reliability can be obtained with an easy and convenient operation.

[Patent document 1] Japanese Laid-Open Patent Publication No. 2005-230120

DISCLOSURE OF THE INVENTION

PROBLEMS TO BE SOLVED BY THE INVENTION

However, informing to the subject in which measurement mode or at which site the measurement result is obtained from has not been disclosed in patent document 1. Thus, the subject might get confused and think as if the body composition calculated using an impedance of a different measurement mode or a different site is the body composition calculated using the impedance measured in the same condition.

In view of solving such problem, the present invention aims to provide a body composition measuring instrument enabling the subject to easily recognize the location of the body site to be detected of the potential difference used in calculating the body composition of the whole body.

A body composition measuring instrument according to one aspect of the invention includes a plurality of hand electrodes and a plurality of foot electrodes; a detecting section for detecting a plurality of potential differences at each of a plurality of body sites including a whole body, both hands, and both feet of a subject by using the electrodes on hands and the electrodes on feet; a body composition calculating unit for calculating a body composition of the whole body of the subject based on at least one of the potential differences detected by the detecting section and body information of the subject; and an informing unit for informing information related to the body site to be detected of the potential difference used in calculating the body composition of the whole body.

The term "body composition of the whole body" is at least the fat free mass of the whole body, and is more preferably biological information including muscle mass, bone mass, body fat mass, body fat percentage, muscle percentage, and visceral fat level in addition to the fat free mass.

The term "body site" includes at least the whole body (both hands-both feet), both hands (right hand-left hand), and both feet (right foot-left foot), and more preferably includes one hand-one foot (e.g., right hand-right foot, right hand-left foot etc.) in addition to the whole body, both hands, and both feet.

Preferably, the body composition measuring instrument further includes a determining unit for determining the body site to be detected based on an impedance corresponding to each potential difference and a reference range predetermined for each body site.

Preferably, the body composition measuring instrument further includes a first unit which is arranged with the hand electrodes, the detecting section, and the body composition calculating unit, and which can be gripped by the subject with both hands; a second unit which is arranged with the foot electrodes and on which both feet of the subject can be placed; a cable for electrically connecting the first unit and the second unit, the cable being removable with respect to the first unit or the second unit; a connection detecting unit for detecting the presence of connection between the cable and the first unit or the second unit; and a determining unit for determining the body site to be detected based on the detection result of the connection detecting unit; wherein the determining unit determines the body site to be detected as the whole body when detected as connected by the connection detecting unit, and determines the body site to be detected as both hands when detected as not connected by the connection detecting unit.

Alternatively, the body composition measuring instrument preferably includes a first unit which is arranged with the hand electrodes and which can be gripped by the subject with both hands; a second unit which is arranged with the foot electrodes and on which both feet of the subject can be placed; the second unit including an accommodating section for accommodating the first unit, and an accommodation detecting unit for detecting whether or not the first unit is accommodated in the accommodating section; a cable for electrically connecting the first unit and the second unit; and a determining unit for determining the body site to be detected based on the detection result of the accommodation detecting unit; wherein the determining unit determines the body site to be detected as both feet when detected as accommodated by the accommodation detecting unit, and determines the body site to be detected as the whole body when detected as not accommodated by the accommodation detecting unit.

Preferably, the body composition calculating unit includes a first calculating unit for calculating a first body composition of the whole body by using a whole body impedance based on the first potential difference in the whole body, a correcting unit for correcting a two limbs impedance based on a second potential difference at the body site other than the whole body, and a second calculating unit for calculating a second body composition of the whole body by using the two limbs impedance corrected by the correcting unit.

Preferably, the first calculating unit calculates the first body composition of the whole body of the subject based on the whole body impedance, the body information of the subject, and a predetermined first estimated equation showing a relationship between the whole body impedance, the body information, and the body composition of the whole body; and the body composition measuring instrument further includes a third calculating unit for calculating a third body composition of the whole body of the subject based on the two limbs impedance based on the second potential difference detected when detecting the first potential difference, the body information of the subject, and a predetermined second estimated equation showing a relationship between the two limbs impedance, the body information, and the body composition of the whole body; a correction value calculating unit for calculating a correction value of the two limbs impedance such that the first body composition of the whole body matches for the third body composition of the whole body; and a storage unit for storing the data of the correction value as correlated information.

It is desirable that the correcting unit corrects the two limbs impedance based on the data of the correction value; and the second calculating unit calculates the second body composition of the whole body of the subject based on the corrected two limbs impedance, the body information of the subject, and the second estimated equation.

Preferably, the first calculating unit calculates the first body composition of the whole body of the subject based on the whole body impedance, the body information of the subject, and a predetermined estimated equation showing a relationship between the whole body impedance, the body information, and the body composition of the whole body; and the body composition measuring instrument further includes a correlation calculating unit for calculating a correlation between the whole body impedance and the two limbs impedance based on the second potential difference detected when detecting the first potential difference; and a storage unit for storing correlation data representing the correlation as correlated information.

It is also preferable that the correcting unit corrects the two limbs impedance based on the correlation data; and the second calculating unit calculates the second body composition of the whole body based on the corrected two limbs impedance, the body information of the subject, and the estimated equation.

Alternatively, it is preferable that the body composition calculating unit includes a first calculating unit for calculating a first body composition of the whole body by using a whole body impedance based on a first potential difference in the whole body; a second calculating unit for calculating a second body composition of the whole body by using a two limbs impedance based on a second potential difference at the body site other than the whole body; and a correcting unit for correcting the calculated second body composition of the whole body based on correlated information representing a relationship between the first body composition of the whole body and the second body composition of the whole body.

Preferably, the first calculating unit calculates the first body composition of the whole body of the subject based on the whole body impedance, the body information of the subject, and a predetermined first estimated equation showing a relationship between the whole body impedance, the body information, and the body composition of the whole body; the second calculating unit calculates the second body composition of the whole body of the subject based on the two limbs impedance, the body information of the subject, and a predetermined second estimated equation showing a relationship between the two limbs impedance, the body information, and the body composition of the whole body; and the body composition measuring instrument further includes a correlation calculating unit for calculating a correlation between the first body composition of the whole body and the second body composition of the whole body based on the second potential difference detected when detecting the first potential difference; and a storage unit for storing correlation data representing the correlation as correlated information.

Preferably, the body composition measuring instrument further includes a display section for displaying calculation results of the body composition of the whole body; wherein the informing unit displays information related to the body site to be detected on the display section.

Preferably, the body composition measuring instrument further includes a voice output section for outputting voice; wherein the informing unit outputs information related to the body site to be detected to the voice output section by voice.

Preferably, the body composition measuring instrument further includes a storage unit for storing the calculated body composition of the whole body and the information related to the body site to be detected in correspondence to each other.

Preferably, the body composition measuring instrument further includes a readout section for reading out the body composition of the whole body stored in the storage unit; wherein the informing unit simultaneously informs the read out body composition of the whole body and the information related to the body site to be detected stored in correspondence to the body composition of the whole body.

EFFECT OF THE INVENTION

According to the present invention, the subject is able to easily recognize based on what potential difference at which body site the body composition of the whole body is calculated. Thus, mix up like a result calculated based on the potential difference at the same body site can be thereby prevented.

BRIEF DESCRIPTION OF THE DRAWINGS

FIG. 1 is a view showing one example of an outer appearance of a body composition measuring instrument according to first to third embodiments of the present invention.

FIG. 2 is a block diagram showing a hardware configuration of the body composition measuring instrument according to the first to the third embodiments of the present invention.

FIG. 3 is a function block diagram of the body composition measuring instrument according to the first embodiment of the present invention.

FIG. 4 is a view showing one example of a data structure of a memory in the body composition measuring instrument according to the first embodiment of the present invention.

FIG. 5 is a flowchart showing a body composition measurement process executed by the control section of the body composition measuring instrument according to the first to the third embodiments of the present invention.

FIG. 6 is a flowchart showing a mode determination process in the first to the third embodiments of the present invention.

FIG. 7 is a flowchart showing another example of the mode determination process in the first to the third embodiments of the present invention.

FIG. 8 is a flowchart showing the whole body measurement process in the first to the third embodiments of the present invention.

FIG. 9 is a view showing one example of a display screen in step S120 of FIG. 8.

FIG. 10 is a flowchart showing a first setting process in the first embodiment of the present invention.

FIG. 11 is a flowchart showing a second setting process in the first embodiment of the present invention.

FIG. 12 is a flowchart showing the hand measurement process in the first to the third embodiments of the present invention.

FIG. 13 is a flowchart showing a first body composition calculating process in the first embodiment of the present invention.

FIG. 14 is a view showing one example of a display screen in step S314 of FIG. 12.

FIG. 15 is a flowchart showing the hand measurement process in the first to the third embodiments of the present invention.

FIG. 16 is a flowchart showing a second body composition calculating process in the first embodiment of the present invention.

FIG. 17 is a view showing one example of a display screen in step S514 of FIG. 15.

FIG. 18 is a flowchart showing a memory readout/display process according to a variant of the first embodiment of the present invention.

FIG. 19 is a view showing one example of a display screen in step S908 of FIG. 18.

FIG. 20 is a function block diagram of a body composition measuring instrument according to the second embodiment of the present invention.

FIG. 21 is a view showing one example of a data structure of a memory in the body composition measuring instrument of the second embodiment of the present invention.

FIG. 22 is a flowchart showing a first setting process in the second embodiment of the present invention.

FIG. 23 is a flowchart showing a second setting process in the second embodiment of the present invention.

FIG. 24 is a flowchart showing a first body composition calculating process in the second embodiment of the present invention.

FIG. 25 is a flowchart showing a second body composition calculating process in the second embodiment of the present invention.

FIG. 26 is a function block diagram of a body composition measuring instrument according to the third embodiment of the present invention.

FIG. 27 is a view showing one example of a data structure of a memory in the body composition measuring instrument of the third embodiment of the present invention.

FIG. 28 is a flowchart showing a first setting process in the third embodiment of the present invention.

FIG. 29 is a flowchart showing a second setting process in the third embodiment of the present invention.

FIG. 30 is a flowchart showing a first body composition calculating process in the third embodiment of the present invention.

FIG. 31 is a flowchart showing a second body composition calculating process in the third embodiment of the present invention.

| 1 | upper limb unit |
|---|---|
| 2 | lower limb unit |
| 3 | cable |
| 10a | body unit |
| 10b, 10c | grip |
| 11 | detecting section |
| 12, 12A, 12B | control section |
| 13 | timer |
| 14 | memory |
| 15 | display section |
| 16 | operation section |
| 17 | power section |
| 18, 31 | connector |
| 19 | sensor |
| 20 | accommodating section |
| 21 | accommodation detecting unit |
| 22 | weight measurement section |
| 100 | body composition measuring instrument |
| 101 | while body impedance measuring unit |
| 102 | two limbs impedance measuring unit |
| 103 | first body composition calculating unit |
| 104, 205, 304 | correcting unit |
| 105, 204 | second body composition calculating unit |
| 105 | correlation setting unit |
| 107 | determining unit |
| 108 | informing unit |
| 206, 306 | correlation calculating unit |
| 1061 | third body composition calculating unit |
| 1062 | correction value calculating unit |
| E10 | hand electrodes |
| E20 | foot electrodes |
| E11, E12, E13, E14, E21, E22, E23, E24 | electrode |

BEST MODE FOR CARRYING OUT THE INVENTION

The embodiments of the present invention will be described in detail with reference to the drawings. Same reference numerals are denoted for the same or corresponding components throughout the drawings.

[First embodiment]

<Outer appearance and configuration of body composition measuring instrument according to first embodiment of the present invention>

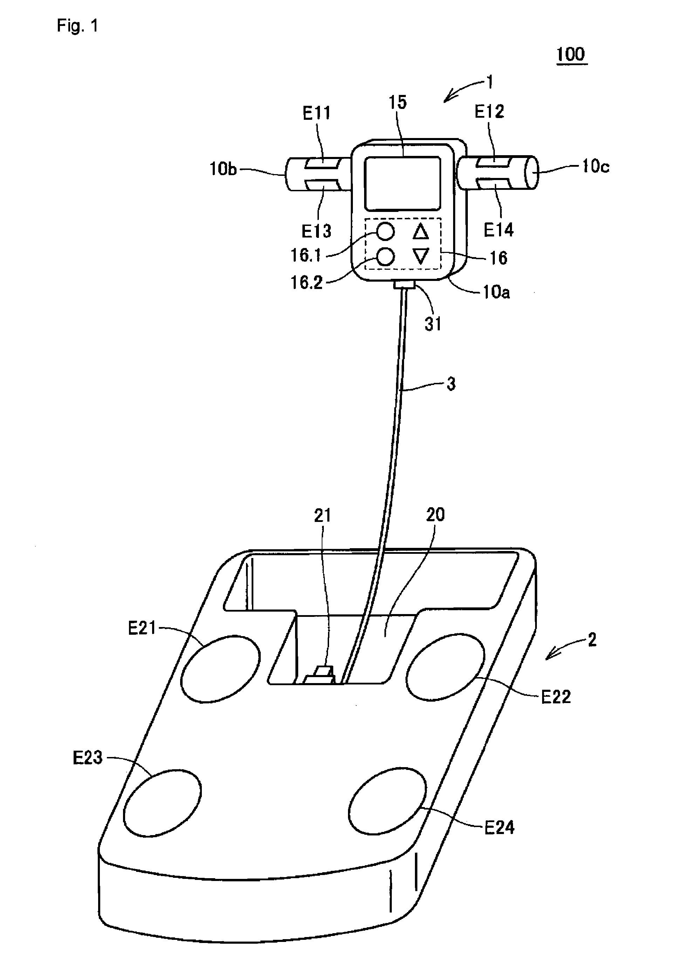

FIG. 1 is a view showing one example of an outer appearance of a body composition measuring instrument 100 according to the first embodiment of the present invention.

With reference to FIG. 1, the body composition measuring instrument 100 is configured including an upper limb unit 1 that can be gripped by the subject with both hands, a lower limb unit 2 on which both feet of the subject can be placed, and a cable 3 for electrically connecting the upper limb unit 1 and the lower limb unit 2.

The upper limb unit 1 includes a main body 10a, and grips 10b, 10c arranged on the left and the right of the main body 10a. The main body 10a is arranged with a display section 15 for displaying the measurement result and various information, and an operation section 16 operated by the subject to accept instruction and input of various information from the subject. A plurality of electrodes E11, E12, E13, and E14 are arranged on the grips 10b, 10c. The grips 10b, 10c are configured so as to be gripped by the subject with both hands. The electrodes E11, E13 are arranged on the grip 10b for the left hand, and the electrodes E12, E14 are arranged on the grip 10c for the right hand. The electrodes E11, E12 arranged on the upper side (head side of the subject in the measuring pose) of the respective grips 10b, 10c are current application electrodes, and the electrodes E13, E14 arranged on the lower side of the respective grips 10b, 10c are voltage detection electrodes. Here, description is made such that the upper limb unit 1 includes the grips 10b, 10c configured in a handle shape, but not limited to such configuration. It is preferable that the subject is able to grip the upper limb unit 1 with both hands and the electrodes E11 to E14 are arranged at the portion to be gripped with both hands. That is, it is preferable that the electrodes E11, E13 are contact with the left hand of the subject and the electrodes E12, E14 are contact with the right hand of the subject.

A plurality of electrodes E21, E22, E23, and E24 are arranged on the upper surface (face on which both feet of the subject are placed) of the lower limb unit 2. The electrodes E21, E22 arranged on the front side (toe side of the subject in the measuring pose) of the lower limb unit 2 are current application electrodes, and the electrodes E23, E24 arranged on the back side (heel side of the subject in the measuring pose) of the lower limb unit 2 are voltage detection electrodes. The lower limb unit 2 includes an accommodating section 20 for accommodating the upper limb unit 1. Furthermore, an accommodation detecting unit 21 for detecting the accommodation of the upper limb unit 1 in the accommodating section 20 is preferably arranged in the lower limb unit 2. The accommodation detecting unit 21 is configured by a sensor and the like.

A connector 31 enabling the attachment of a connector 18 built in the upper limb unit 1 is preferably arranged at the end of the cable 3. The upper limb unit 1 and the cable 3 are removably attachable in the present embodiment, but the lower limb unit 2 and the cable 3 may be removably attachable.

In the following description, the electrodes E11 to E14 are collectively termed as "hand electrodes E10" and the electrodes E21 to E24 are collectively termed as "foot electrodes E20".

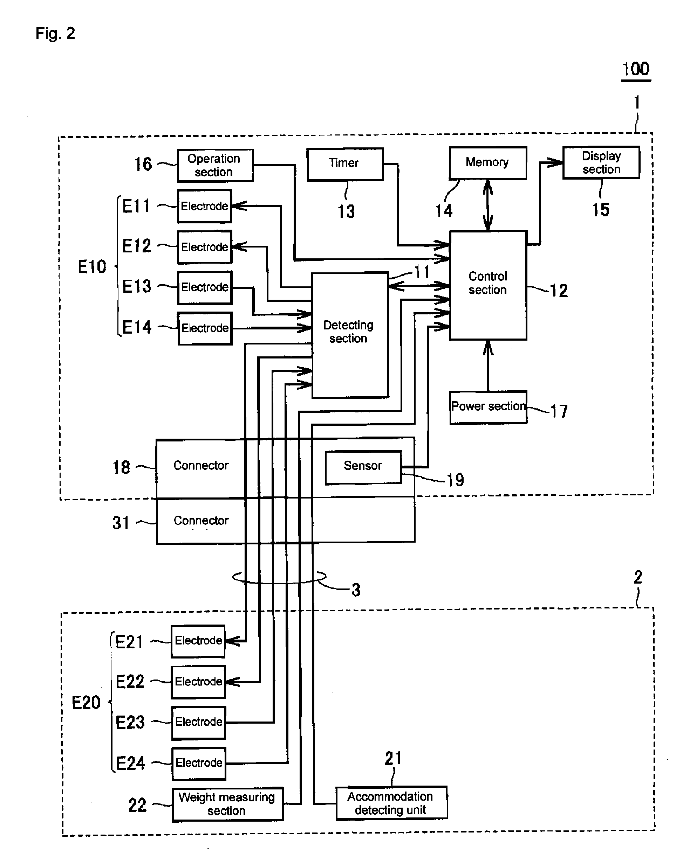

FIG. 2 is a block diagram showing a hardware configuration of a body composition measuring instrument 100 according to the first embodiment of the present invention.

In addition to the hand electrodes E10, the display section 15, the operation section 16, and the connector 18 described above, the upper limb unit 1 further includes a detecting section 11 for detecting the potential difference between the hand and the foot (whole body) by applying current between the hands and the feet with both the hand electrodes E10 and the foot electrodes E20, and detecting a plurality of potential differences at a plurality of body sites including the whole body (both hands-both feet), both hands (right hand-left hand), and both feet (right foot-left foot) of the subject with one of the hand electrodes E10 or the foot electrodes E20; a control section 12 for controlling the entire body composition measuring instrument 100; a timer 13 for measuring date and time; a memory 14 for storing various data and programs; a power unit 17 for supplying power to the control section 12; and a sensor 19 for detecting attachment and detachment of the cable 3 and the upper limb unit 1.

The detecting section 11 changes over the electrodes when controlled by the control section 12. The information on the detected potential difference is output to the control section 12. The detecting section 11 is connected to, for example, all the hand electrodes E10 and the foot electrodes E20. The detecting section 11 includes a changing-over switch (not shown) for changing over the electrode according to the instruction from the control section 12, and a constant current generating unit (not shown) for flowing constant current to at least one pair of current electrodes selected by the changing-over switch, wherein the potential difference of at least one pair of voltage electrodes selected by the changing-over switch is detected while the constant current is applied to the subject through the current electrodes.

In the following description, the impedance based on the potential difference detected by the detecting section 11 by using both the hand electrodes E10 and the foot electrodes E20 is referred to as "whole body impedance". The impedance based on the potential difference detected by the detecting section 11 by using only the hand electrodes E10 is referred to as "impedance between both hands", and the impedance based on the potential difference detected by the detecting section 11 by using only the foot electrodes E20 is referred to as "impedance between both feet". The impedance at body sites (both hands, both feet, right hand-left foot, etc.) other than the whole body such as impedance between both hands and impedance between both feet is referred to as "two limbs impedance".

The control section 12 is configured by a CPU (Central Processing Unit) and the like. The memory 14 is configured by a nonvolatile memory such as a flash memory. The display section 15 is configured by liquid crystal and the like. The operation section 16 includes a power switch 16.1 for inputting the instruction of ON/OFF of the power, a measurement start switch 16.2 for instructing the start of measurement, and the like.

In addition to the foot electrodes E20 and the accommodation detecting unit 21 described above, the lower limb unit 2 also desirably includes a weight measurement section 22 for measuring the weight of the subject. The weight measurement section 22 is configured by a sensor and the like.

The body composition measuring instrument 100 according to the present embodiment is a device for measuring the body composition of the whole body of the subject. The body composition measuring instrument 100 has a "whole body measurement mode" for measuring the body composition of the whole body based on the whole body impedance (expressed as "Zw"), and a "simple measurement mode" for measuring the body composition of the whole body based on the two limbs impedance, that is, impedance between both hands (expressed as "Zh") or impedance between both feet (expressed as "Zf"). The simple measurement mode includes "hand-simple measurement mode" for measuring the body composition of the whole body based on the impedance between both hands Zh, and "foot-simple measurement mode" for measuring the body composition of the whole body based on the impedance between both feet Zf.

The measuring pose of the subject when measuring the body composition of the whole body in the whole body measurement mode is a state in which both hands and both feet of the subject are contact with the hand electrodes E10 and the foot electrodes E20, respectively. The measuring pose of the subject when measuring the body composition of the whole body in the hand-simple measurement mode is a state in which both hands of the subject are contact with the hand electrodes E10. The measuring pose of the subject when measuring the body composition of the whole body in the foot-simple measurement mode is a state in which both feet of the subject are contact with the foot electrodes E10.

The measurement can be easily and conveniently made when measuring the body composition of the whole body in the simple measurement mode, but the reliability in the calculation result of the body composition might be poor due to influence of daily fluctuation. Therefore, in the first embodiment of the present invention, information related to the body site (hereinafter referred to as "measurement site") to be detected of the potential difference used in the calculation of the body composition of the whole body is informed to the subject. As used herein, "information related to measurement site" may be information representing the measurement site itself or information indirectly representing the measurement site such as mode name. The mix up like the body composition calculated based on the potential difference at the same measurement site can be thereby prevented.

However, the subject demands to obtain a result of body composition of as high as possible reliability. Therefore, even in the simple measurement mode, a correction process may be performed to calculate the body composition having high reliability similar to the measurement in the whole body measurement mode. The subject then further obtains information related to the measurement site while obtaining the body composition having high reliability, so that whether or not the value of the body composition is a value having a possibility of being corrected can be even known.

In the present embodiment, description is made as performing the correction process based on the correlated information to be hereinafter described in the case of the simple measurement mode.

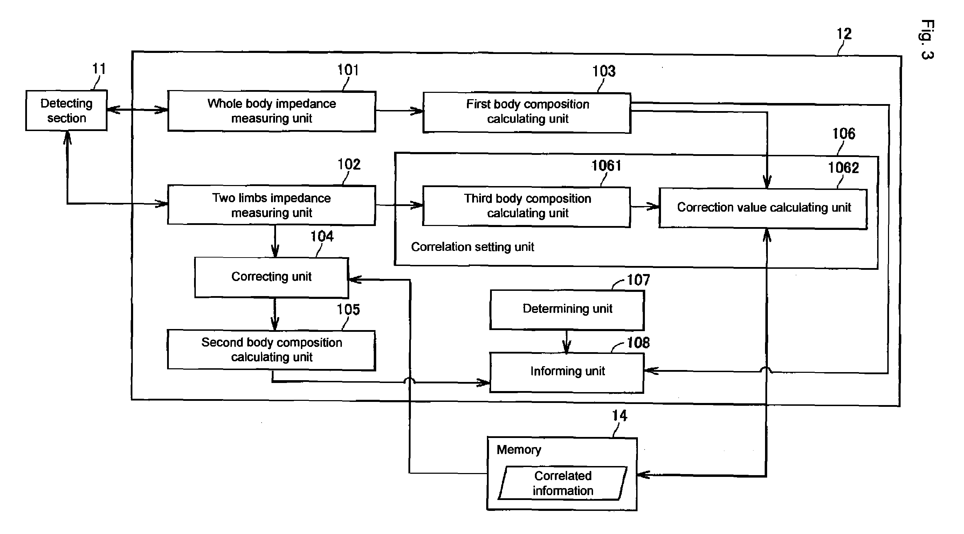

FIG. 3 is a function block diagram of the body composition measuring instrument 100 according to the first embodiment of the present invention.

With reference to FIG. 3, the control section 12 includes a whole body impedance measuring unit 101 for measuring the whole body impedance, a two limbs impedance measuring unit 102 for measuring the two limbs impedance, a first body composition calculating unit 103 for calculating the body composition of the whole body based on the whole body impedance measured by the whole body impedance measuring unit 101, a correcting unit 104 for correcting the two limbs impedance measured by the two limbs impedance measuring unit 102, a second body composition calculating unit 105 for calculating the body composition of the whole body based on the two limbs impedance after being corrected by the correcting unit 104, a correlation setting unit 106 for setting the correlated information, a determining unit 107 for determining the measurement site, and an informing unit 108 for informing information related to the measurement site determined by the determining unit 107.

The correlated information is data of the corrected value of the two limbs impedance in the first embodiment of the present invention.

The whole body impedance measuring unit 101 controls the detecting section 11 and measures the whole body impedance in the whole body measurement mode. Specifically, in a state that the current flows from the electrodes E11, E12 to the electrodes E21, E22 and the current is applied to the whole body of the subject, a control for detecting the potential difference (hereinafter referred to as "whole body potential difference") between the electrodes E13, E14 and the electrodes E23, E24 is performed. The whole body impedance Zw is calculated (measured) based on the whole body potential difference detected in this manner. When measuring the whole body impedance, it is preferable that the electrode E11 and the electrode E12, the electrode E21 and the electrode E22, the electrode E13 and the electrode E14, and the electrode E23 and the electrode E24 are respectively short circuit.

The two limbs impedance measuring unit 102 controls the detecting section 11 and measures the two limbs impedance in each of the whole body measurement mode and the simple measurement mode. In the whole body measurement mode, the impedance between both hands Zh and the impedance between both feet Zf are measured. The impedance between both hands Zh is measured in the hand-simple measurement mode, and the impedance between both feet Zf is measured in the foot-simple measurement mode. When measuring the impedance between both hands Zh, the two limbs impedance measuring unit 102 specifically performs a control for detecting the potential difference (hereinafter referred to as "potential difference between both hands") between the electrode E13 and the electrode E14 in a state that the current is flowed between the electrode E11 and the electrode E12 and the current is applied between the hands of the subject. When measuring the impedance between both feet Zf, the two limbs impedance measuring unit 102 specifically performs a control for detecting the potential difference (hereinafter referred to as "potential difference between both feet") between the electrode E23 and the electrode E24 in a state that the current is flowed between the electrode E21 and the electrode E22 and the current is applied between the feet of the subject.

The first body composition calculating unit 103 and the second body composition calculating unit 105 calculate body fat percentage etc. as the body composition of the whole body, respectively. The body fat percentage (% FAT) is calculated using the following equation (1).

$$\%FAT = W\text{-}FFM/W*100 \qquad (1)$$

(FFM: fat free mass, W: weight)

The estimated equation of the fat free mass FFM (of the whole body) is set in advance for when using the whole impedance Za, when using the impedance between both hands Zh, and when using the impedance between both feet Zf. That is, the fat free mass of the subject is calculated using the following estimated equations (2) to (4) representing the relationship between each impedance, body information, and fat free mass defined in advance by the correlation with the reference measured by MRI etc. The fat free mass estimated using the whole body impedance Zw is expressed as "FFM_w", the fat free mass estimated using the impedance between both hands Zh is expressed as "FFM_h", and the fat free mass estimated using the impedance between both feet Zf is expressed as "FFM_Zf".

$$FFM\_w = \alpha_1*H^2/Zw + \beta_1*W + \gamma_1 \qquad (2)$$

$$FFM\_h = \alpha_2*H^2/Zh + \beta_2*W + \gamma_2 \qquad (3)$$

$$FFM\_f = \alpha_3*H^2/Zf + \beta_3*W + \gamma_3 \qquad (4)$$

(wherein $\alpha_1$, $\beta_1$, $y_1$, $\alpha_2$, $\beta_2$, $\gamma_2$, $\alpha_3$, $\beta_3$, $\gamma_3$: coefficient, H: height, W: weight)

The coefficient in the estimated equation may differ depending on the attribute (age and sex) of individual.

As described above, the first body composition calculating unit 103 calculates the body composition (body fat percentage) of the whole body of the subject based on the whole body impedance Zw measured by the whole body impedance measuring unit 101, the body information of the subject, and the equations (1) and (2). The second body composition calculating unit 105 calculates the body composition of the whole body of the subject based on the corrected impedance between both hands (expressed as "Zh'") by the correcting unit 104, the body information of the subject, and the equations (1) and (3) in the hand-simple measurement mode. The second body composition calculating unit 105 calculates the body composition of the whole body of the subject based on the corrected impedance between both feet (expressed as "Zf'") by the correcting unit 104, the body information of the subject, and the equations (1) and (4) in the foot-simple measurement mode. In the present embodiment, the body composition of the whole body is calculated based on each impedance value and the body information, as shown in the estimated equations (2) to (4), but the body composition of the whole body may be calculated based on the value of each potential difference and the body information.

The correcting unit 104 corrects the two limbs impedance measured by the two limbs impedance measuring unit 102 based on the correlated information (data of correction value of the two limbs impedance) stored in the memory 14 in the simple measurement mode.

The correlation setting unit 106 includes a third body composition calculating unit 1061 for calculating the body composition of the whole body based on the two limbs impedance measured by the two limbs impedance measuring unit 102 in the whole body measurement mode, and a correction value calculating unit 1062 for calculating the correction value of the two limbs impedance so that the body composition of the whole body calculated by the first body composition calculating unit 103 matches for the body composition of the whole body calculated by the third body composition calculating unit 1061. Specifically, the third body composition calculating unit 1061 calculates the body composition of the whole body of the subject based on the impedance between both hands Zh based on the potential difference between both hands detected when detecting the potential difference of the whole body, the body information of the subject, and the equations (1) and (3). Furthermore, the body composition of the whole body of the subject is calculated based on the impedance between both feet Zf based on the potential difference between both feet detected when detecting the potential difference of the whole body, the body information of the subject, and the equations (1) and (4). As used herein, the phrase "when detecting the potential difference of the whole body" merely needs to be at least within a period of a series of measurement process in the whole body measurement mode.

The determining unit 107 determines, for instance, what mode the measurement mode corresponding to the measurement site is. That is, determination is made on which measurement of the whole body measurement mode, the hand-simple measurement mode, or the foot-simple measurement mode is to be executed. The specific determination method will be described below.

The informing unit 108 preferably informs the information related to the determined measurement site along with the information on the body composition of the whole body. Specifically, the informing unit 108 performs a process of displaying the information related to the measurement site along with the information on the body composition of the whole body on the display section 15. The information related to the measurement site includes those in which data (e.g., data such as character, picture, symbol, and the like) representing each measurement site is stored in advance in the memory 14. The informing unit 108 reads out the data corresponding to the determined measurement site and displays the same. Here, the informing unit 108 displays the information related to the measurement site on the display section 15, but the informing manner is not limited thereto. For instance, information related to the measurement site may be output by voice at the voice output section such as speaker (not shown). In this case, the informing unit 108 displays the body composition of the whole body on the display section 15, and outputs the name of the measurement site by voice, for example, while displaying the body composition of the whole body. Alternatively, a melody that differs for every measurement site may be output.

The control section 12 preferably determines the time zone (e.g., morning time zone, afternoon time zone, night time zone, and the like) of when detecting each potential difference. That is, in the case of the whole body measurement mode, the control section 12 determines the time zone of when detecting the potential difference of the whole body based on the timed data from the timer 13. In the case of the simple measurement mode, the control section 12 determines the time zone of when detecting the potential difference between both hands or when detecting the potential difference between both feet based on the timed data from the timer 13. As used herein, the phrase "when detecting the potential difference between both hands or when detecting the potential difference between both feet" merely needs to be at least within a period of a series of measurement process in the simple measurement mode.

The operation of each function block may be realized by executing software stored in the memory 14, or may be realized by hardware for at least one part.

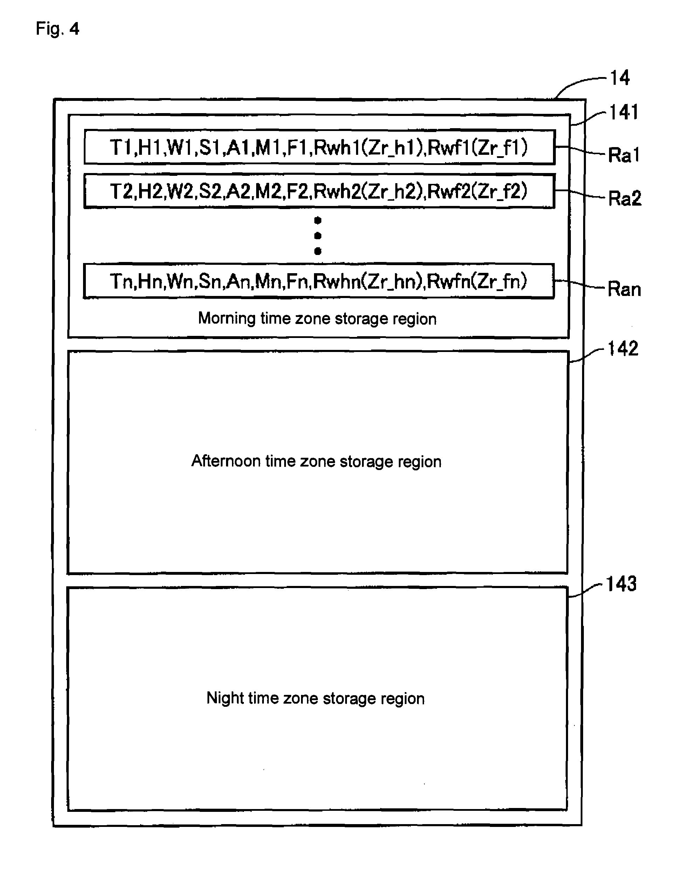

FIG. 4 is a view showing one example of a data structure of the memory 14 in the body composition measuring instrument 100 according to the first embodiment of the present invention.

With reference to FIG. 4, the memory 14 includes a morning time zone storage region 141 for storing the measurement result in the morning time zone, an afternoon time zone storage region 142 for storing the measurement result in the afternoon time zone, and a night time zone storage region 143 for storing the measurement result in the night time zone. Among such storage regions, which region to store the measurement result is determined according to the time zone determined by the control section 12. The range of the time zone may be defined in advance at the time of shipping, or may be set by the user according to his/her life cycle. For instance, "morning time zone" may be defined as between five to ten o'clock, "afternoon time zone" may be between ten to sixteen o'clock, and "night time zone" may be between sixteen and four o'clock the following day.

When the body composition measurement process to be described in detail below is executed, the measurement result is stored in the memory 14 in the storage region corresponding to the time zone in measurement in units of records Ra. The record Ra (Ra1, Ra2,..., Ran) includes date and time data T in measurement (in detection of each potential difference), height input value data H serving as body information, weight value data W serving as body information, sex data S serving as body information, age data A serving as body information, measurement mode data M, body composition data F of the whole body serving as measurement result, correlated information Rwh, and correlated information Rwf. Such data merely need to be stored in each region in association to each other for every measurement, and is not limited to a storage form using record Ra. The storage region is arranged in advance for every time zone, but the storage region may not be arranged for every time zone. For instance, identification data indicating the time zone may be included in the record Ra, so that result is stored in the memory 14 in the order of measurement date and time.

The measurement mode data M is information related to the measurement site, and specifically, is identification information indicating which measurement mode of the whole body measurement mode, the hand-simple measurement mode, and the foot-simple measurement mode is executed. For instance, "0" is stored for the whole body measurement mode, "1" is stored for the hand-simple measurement mode, and "2" is stored for the foot-simple measurement mode.

The body composition data F of the whole body is the measurement result of the final body composition, and is the data of the body fat percentage calculated by the first body composition calculating unit 103 or the second body composition calculating unit 105.

The correlated information Rwh has the data of the correction value Zr_h of the impedance between both hands Zh stored in the present embodiment.

The correlated information Rwf has the data of the correlation value Zr_f of the impedance between both feet Zf stored in the present embodiment.

When the body composition of the whole body is measured in the whole body measurement mode, all the data described above are stored in the memory 14. When the body composition of the whole body is measured in the hand-simple measurement mode, data other than the weight W, the correlated information Rwh, and the correlated information Rwf are stored in the memory 14. Furthermore, when the body composition of the whole body is measured in the foot-simple measurement mode, data other than the correlated information Rwh and the correlated information Rwf are stored in the memory 14.

<Operation of body composition measuring instrument according to the first embodiment of the present invention>

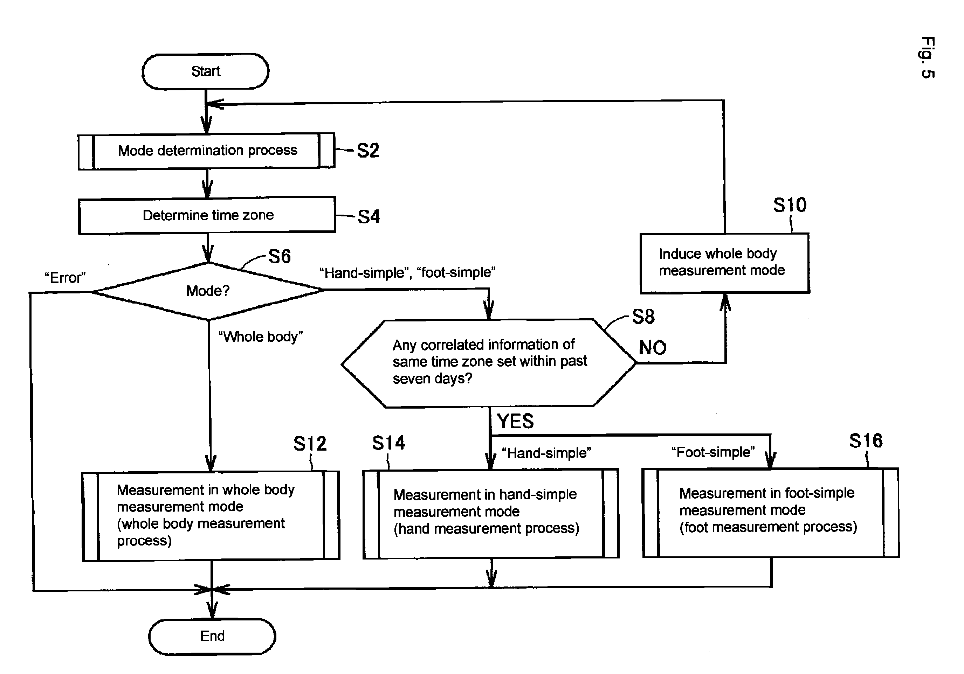

FIG. 5 is a flowchart showing a body composition measurement process executed by the control section 12 of the body composition measuring instrument 100 according to the first embodiment of the present invention. The processes shown in the flowchart of FIG. 5 are stored in the memory 14 in advance as a program, wherein the function of the body composition measurement process is realized when the control section 12 reads out and executes the relevant program. The processes described below are started, for instance, when the measurement start switch 16.2 is pushed.

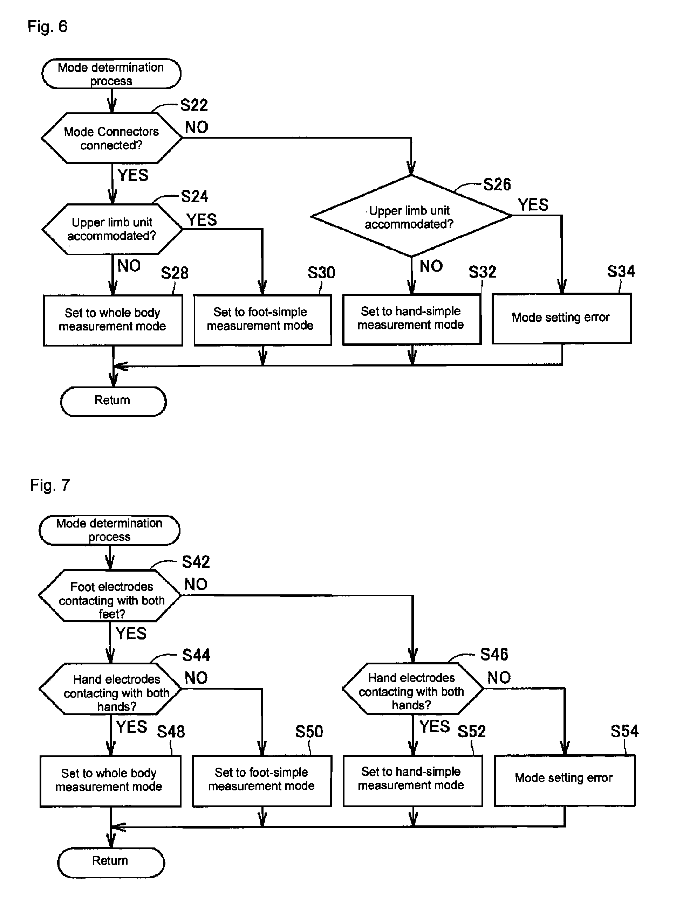

With reference to FIG. 5, the determining unit 107 performs a mode determination process (step S2). The sub-routines of the mode determination process in step S2 are shown in FIG. 6.

With reference to FIG. 6, the determining unit 107 determines whether or not the connector 18 and the connector 31 are connected based on a signal from the sensor 19 (step S22). That is, whether or not the upper limb unit 1 and the cable 3 are connected is determined. If determined that the connector 18 and the connector 31 are connected (YES in step S22), the process proceeds to step S24. If determined that the connector 18 and the connector 31 are not connected (NO in step S22), the process proceeds to step S26.

In step S24, the determining unit 107 determines whether or not the upper limb unit 1 is accommodated in the accommodating section 20 based on a signal from the accommodation detecting unit 21. If determined that the upper limb unit 1 is not accommodated in the accommodating section 20 (NO in step S24), the determining unit 107 determines that the measurement site is the whole body, and sets the subsequent measurement process to the whole body measurement mode (step S28). If determined that the upper limb unit 1 is accommodated in the accommodating section 20 (YES in step S24), the determining unit 107 determines that the measurement site is both feet, and sets the subsequent measurement process to the foot-simple measurement mode (step S30).

In step S26 as well, the determining unit 107 determines whether or not the upper limb unit 1 is accommodated in the accommodating section 20 based on a signal from the accommodation detecting unit 21. If determined that the upper limb unit 1 is not accommodated in the accommodating section 20 (NO in step S26), the determining unit 107 determines that the measurement site is both hands, and sets the subsequent measurement process to the hand-simple measurement mode (step S32). If determined that the upper limb unit 1 is accommodated in the accommodating section 20 (YES in step S26), determination is made as mode setting error (determination of measurement site is not possible) (step S34). The mode determination process is then terminated.

The subject thus merely takes a measuring pose of each measurement mode, so the measurement site is automatically determined and the measurement corresponding to each measurement mode is started.

Again referring to FIG. 5, the control section 12 determines the time zone in measurement based on the output data from the timer 13 (step S4).

The control section 12 then determines the mode determined in step S2 (step S6). If in the whole body measurement mode, the measurement process (whole body measurement process) in the whole body measurement mode is executed (step S12). If in the hand-simple measurement mode or the foot-simple measurement mode, the control section 12 determines whether or not there is correlated information of the same time zone set in the past, for example, within seven days (step S8). If determined that there is correlated information of the same time zone set within the past seven days (YES in step S8), the process proceeds to step S14 or step S16. That is, if the mode determined in step S2 is the hand-simple measurement mode, the process proceeds to step S14. If the mode determined in step S2 is the foot-simple measurement mode, the process proceeds to step S16. In the present embodiment, "same time zone" refers to the same time zone as the time zone determined in step S4 (i.e., time zone in the measurement for this time).

The measurement process (hand measurement process) in the hand-simple measurement mode is executed in step S14. The measurement process (foot measurement process) in the foot-simple measurement mode is executed in step S16.

If determined that there is no correlated information of the same time zone set within the past seven days in step S8 (NO in step S8), the control section 12 induces the measurement in the whole body measurement mode (step S10). Specifically, for example, the control section 12 performs a process of displaying a message "please measure in the whole body measurement mode" on the display section 15.

In the above description, the mode, that is, the measurement site is determined based on the signals from the sensor 19 and the accommodation detecting unit 21, but is not limited to such method. A button corresponding to each mode (measurement site) may be arranged on the operation section 16, so that the subject can select which mode to execute (with which measurement site to perform the measurement). Alternatively, the mode determination process shown in FIG. 7 may be performed.

FIG. 7 is a flowchart showing another example of the mode determination process in the first embodiment of the present invention. With reference to FIG. 7, the determining unit 107 determines whether or not the foot electrodes E20 are contact with both feet of the subject (step S42). If determined that the foot electrodes E20 are contact with both feet of the subject (YES in step S42), the process proceeds to step S44.

If determined that the foot electrodes E20 are not contact with both feet of the subject (NO in step S42), the process proceeds to step S46.

In step S44, the determining unit 107 determines whether or not the hand electrodes E10 are contact with both hands of the subject. If determined that the hand electrodes E10 are contact with both hands of the subject (YES in step S44), the determining unit 107 determines that the measurement site is the whole body, and sets the subsequent measurement process to the whole body measurement mode (step S48). If determined that the hand electrodes E10 are not contact with both hands of the subject (No in step S44), the determining unit 107 determines that the measurement site is both feet, and sets the subsequent measurement process to the foot-simple measurement mode (step S50).

In step S46 as well, the determining unit 107 determines whether or not the hand electrodes E10 are contact with both hands of the subject. If determined that the hand electrodes E10 are contact with both hands of the subject (YES in step S46), the determining unit 107 determines that the measurement site is both hands, and sets the subsequent measurement process to the hand-simple measurement mode (step S52). If determined that the hand electrodes E10 are not contact with both hands of the subject (NO in step S46), determination is made as mode setting error (determination of measurement site is not possible) (step S54). The mode determination process is then terminated.

The determinations in steps S42, 44, and 46 can be realized by using the method disclosed in patent document 1 (Japanese Laid-Open Patent Publication No. 2005-230120). More specifically, the contacting state can be determined by comparing the impedance based on the potential difference at each body site (whole body, both hands, both feet) and the reference range defined in advance for each body site. In the flowchart of FIG. 7, determination is made on whether the measurement site is the whole body, both hands, or both feet, but in addition to such sites, determination may be made for right hand-right foot, right hand-left foot, left hand-left foot, and left hand-right foot. In other words, one hand-one foot mode can be further provided in addition to the whole body measurement mode and the simple measurement mode. In this case, if determined that the hand electrodes is not contact with both hands in step S46 (NO in step S46), contacting state to the electrodes of other body sites (right hand-right foot, right hand-left foot, left hand-left foot, and left hand-right foot) is further detected to determine the measurement site.

The whole body measurement process (S12), the hand measurement process (S14), and the foot measurement process (S16) shown in FIG. 5 are respectively described below with sub-routines.

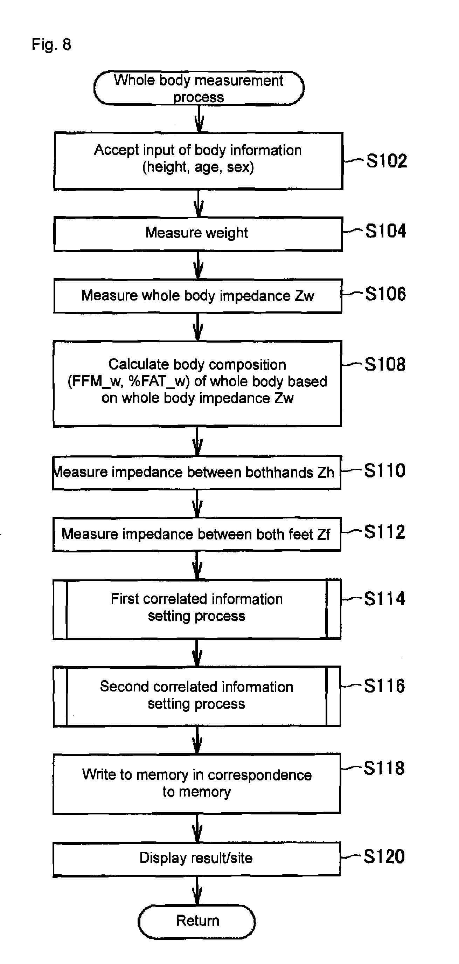

FIG. 8 is a flowchart showing the whole body measurement process in the first embodiment of the present invention. With reference to FIG. 8, the control section 12 accepts the input of body information (height, age, sex) from the subject (step S102). The control section 12 then measures the weight with the weight measurement section 22 (step S104).

The whole body impedance measuring unit 101 measures the whole body impedance Zw of the subject (step S106). Subsequently, the first body composition calculating unit 103 calculates the body composition of the whole body, that is, body fat percentage (expressed as "%FAT_w") based on the whole body impedance Zw measured in step S106 (step S108). More specifically, the first body composition calculating unit 103 first calculates the fat free mass (FFM_w) of the whole body by using the whole body impedance Zw, the body information of the subject, and the estimated equation (2). The body fat percentage (%FAT_w) is then calculated using equation (1). In the present embodiment, the body fat percentage is calculated after calculating the fat free mass, but the body fat percentage may be directly calculated based on the whole body impedance Zw and the body information of the subject. Alternatively, only the fat free mass may be calculated.

The two limbs impedance measuring unit 102 then measures the impedance between both hands Zh of the subject (step S110). The impedance between both feet Zf of the subject is then measured (S112). The correlation setting unit 106 then performs a first correlated information setting process (step S114) and a second correlated information setting process (step S116). The details of such setting processes will be hereinafter described.

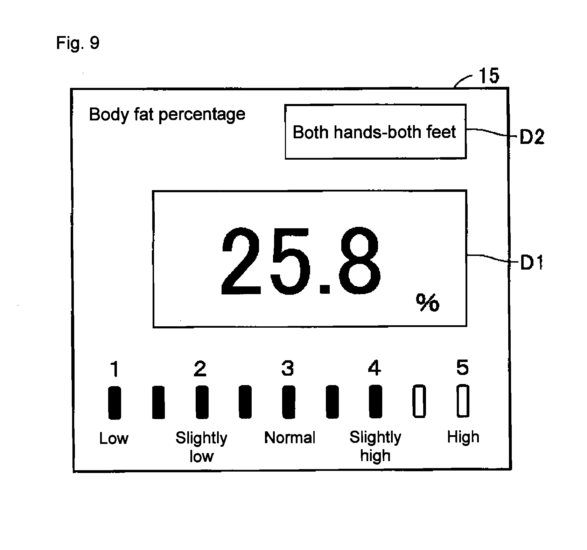

The control section 12 then writes the measurement result, the correlated information, and the like to the memory 14 in correspondence to the time zone determined in step S4 (step S118). The informing unit 108 displays the measurement result (body fat percentage) and the information related to the measurement site on the display section 15 (step S120). One example of a display screen in step S120 is shown in FIG. 9.

With reference to FIG. 9, the body fat percentage of the whole body calculated in step S108 is displayed in a display region D1, and characters "both hands-both feet" etc. are displayed as information related to the measurement site in a display region D2 in the display section 15. The subject is then able to recognize the body fat percentage as being calculated based on the detection result of the potential difference in the whole body (both hands-both feet). That is, the subject can recognize that it is the most reliable body fat percentage. The information of the measurement site itself is displayed as the information related to the measurement site, but information representing the mode such as "whole body measurement mode" may be displayed. In this case as well, the subject is able to similarly recognize the body fat percentage as being calculated based on the detection result of the potential difference in the whole body.

As shown in FIG. 9, advice information on the evaluation of the calculation result of the body fat percentage may be displayed. In the figure, a plurality of blocks is displayed in a predetermined region, and characters and numbers such as low (1), slightly low (2), normal (3), slightly high (4), and high (5) may be displayed in association with the odd number blocks in a stepwise manner from the left side. The block on the very left side to the block at the position corresponding to the evaluation of the calculation result may be lighting displayed (paint displayed) so that the evaluation of the calculation result can be advised to the subject. Here, blocks from "low (1)" to "slightly high (4)" are lighting displayed. Therefore, the subject then can recognizes that the body fat percentage is slightly higher than normal level. The evaluation of the calculation result can be made using an evaluation table (correspondence table of value of body fat percentage and evaluation) formed in advance for every age and sex. The advice information to be displayed is not limited to the aspect shown in FIG. 9, and may be a message such as "more exercise is recommended".

The whole body measurement process is then terminated.

The first correlated information setting process (S114) and the second correlated information setting process (S116) will now be described in detail.

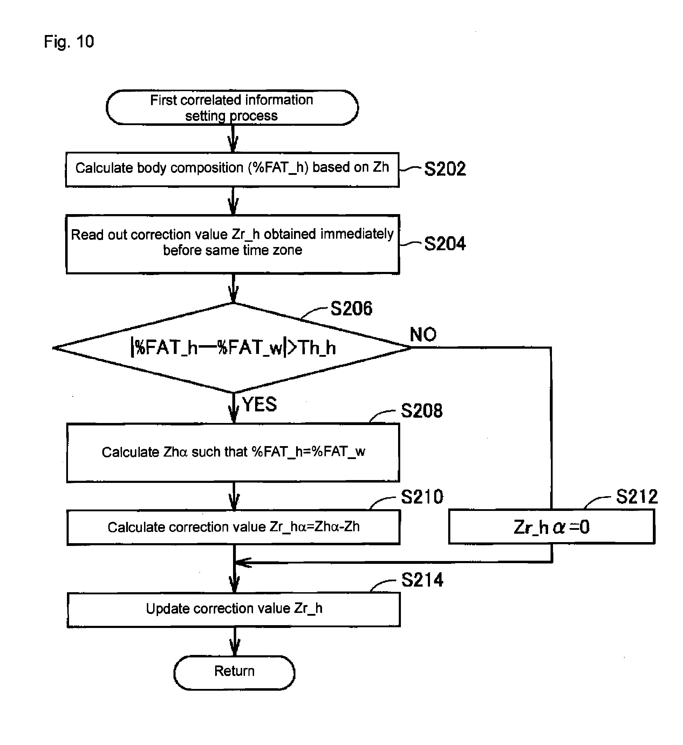

FIG. 10 is a flowchart showing the first correlated information setting process in the first embodiment of the present invention.

With reference to FIG. 10, the third body composition calculating unit 1061 calculates the body composition of the whole body, that is, body fat percentage (expressed as "%FAT_h") based on the impedance between both hands Zh (step S202). More specifically, the third body composition calculating unit 1061 first calculates the fat free mass (FFM_h) of the whole body by using the impedance between both hands Zh, the body information of the subject, and the estimated equation (3). The body fat percentage (%FAT_w) is then calculated. In this case as well, the body fat percentage is calculated after calculating the fat free mass, but the calculating method is not limited thereto.

The correlation setting unit 106 then reads out the data of the correlation value Zr_h or the correlated information immediately before the same time zone from the memory 14 (step S204).

Thereafter, the correlation setting unit 106 determines whether or not a difference value between the body fat percentage %FAT_h calculated in step S202 and the body fat percentage %FAT_w calculated in step S108 exceeds a threshold value Th_h defined in advance (step S206). If determined as exceeding the threshold value Th_h (YES in step S206), the process proceeds to step S208. If determined as not exceeding the threshold value Th_h (NO in step S206), the process proceeds to step S212. The threshold value Th_h is preferably about 0.5% since the difference by daily fluctuation is about 1 %.

In step S208, the correction value calculating unit 1062 calculates an impedance $Zh\alpha$ such that the body fat percentage %FAT_h matches for the body fat percentage %FAT_w. The difference between the impedance $Zh\alpha$ calculated in step S208 and the impedance between both hands Zh measured in step S110 is calculated as a correction value $Zr\_h\alpha$ for this time (step S210). The process proceeds to step S214 after the process of step S210 is terminated.

In step S212, the correction value $Zr\_h\alpha$ for this time is set as "0".

In step S214, the correction value Zr_h is updated. Specifically, for example, a new correction value Zr_h is calculated by averaging the correction value Zr_h immediately before read out in step S204 and the correction value $Zr\_h\alpha$ for this time (e.g., $Zr\_h+Zr\_h\alpha/2$).

If the correlated information of the same time zone is not stored in the memory 14, the correction value $Zr\_h\alpha$ for this time is assumed as the correction value Zr_h.

The first correlated information setting process is then terminated.

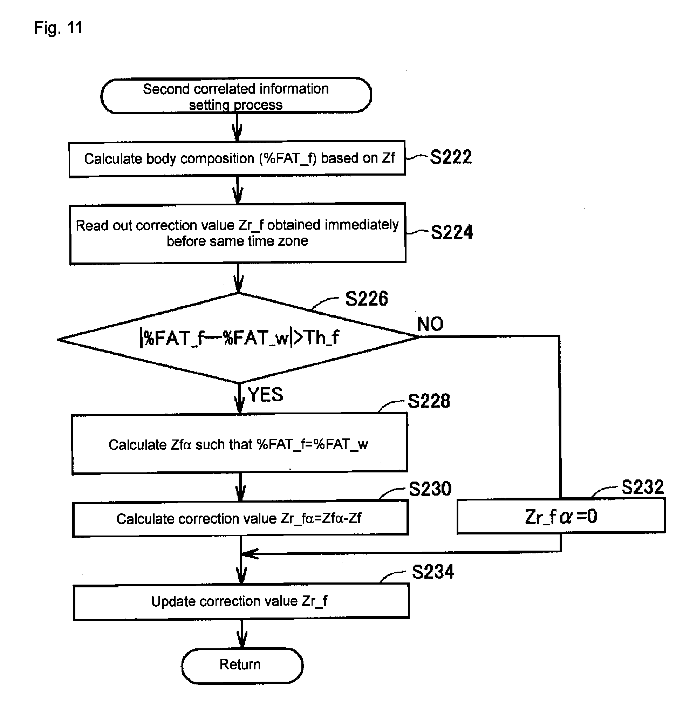

FIG. 11 is a flowchart showing the second correlation setting process in the first embodiment of the present invention.

With reference to FIG. 11, the third body composition calculating unit 1061 calculates the body composition of the whole body, that is, body fat percentage (expressed as "%FAT_f") based on the impedance between both feet Zh (step S222). More specifically, the third body composition calculating unit 1061 first calculates the fat free mass (FFM_f) of the whole body by using the impedance between both feet Zf, the body information of the subject, and the estimated equation (4). The body fat percentage (%FAT_w) is then calculated using equation (1). In this case as well, the body fat percentage is calculated after calculating the fat free mass, but the calculating method is not limited thereto.

The correlation setting unit 106 then reads out the data of the correlation value Zr_f or the correlated information immediately before the same time zone from the memory 14 (step S224).

Thereafter, the correlation setting unit 106 determines whether or not a difference value between the body fat percentage %FAT_f calculated in step S222 and the body fat percentage %FAT_w calculated in step S108 exceeds a threshold value Th defined in advance (step S226). If determined as exceeding the threshold value Th_f (YES in step S226), the process proceeds to step S228. If determined as not exceeding the threshold value Th (NO in step S226), the process proceeds to step S232. The threshold value Th_f is preferably about 0.5% since the difference by daily fluctuation is about 1 %.

In step S228, the correction value calculating unit 1062 calculates an impedance Zfα such that the body fat percentage %FAT_f matches for the body fat percentage %FAT_w. The difference between the impedance Zfα calculated in step S228 and the impedance between both feet Zf measured in step S110 is calculated as a correction value Zr_fα for this time (step S230). The process proceeds to step S234 after the process of step S230 is terminated.

In step S232, the correction value Zr_fα for this time is set as "0".

In step S234, the correction value Zr_f is updated. Specifically, for example, a new correction value Zr_f is calculated by averaging the correction value Zr_f immediately before read out in step S224 and the correction value Zr_fα for this time (e.g., (Zr_f+Zr_fα)/2).

If the correlated information of the same time zone is not stored in the memory 14, the correction value Zr_fα for this time is assumed as the correction value Zr_f.

The second correlated information setting process is then terminated.

In the present embodiment, the correction value is updated by averaging the correction value immediately before and the correction value for this time, but the method is not limited thereto. For instance, all the past correction values may be read out and averaged. Alternatively, the correction values within a predetermined period may be read out and averaged. Alternatively, the correction value for this time may be simply obtained without averaging.

The body fat percentage %FAT_w used in steps S206 and S208 in the first correlated information setting process and in steps S226 and S228 in the second correlated information setting process may be an average value over a constant period of the measurement values in the whole body measurement mode.

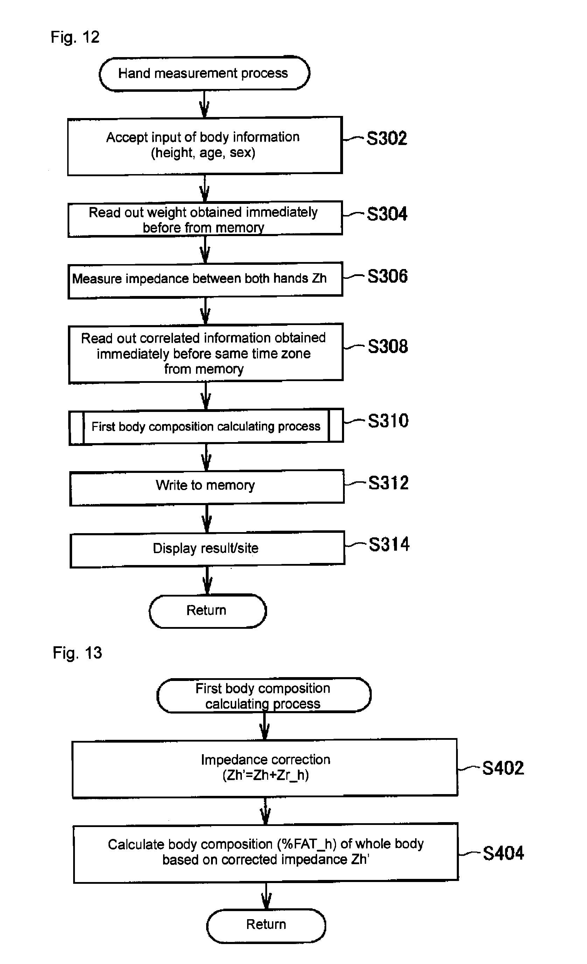

FIG. 12 is a flowchart showing the hand measurement process in the first embodiment of the present invention. With reference to FIG. 12, the control section 12 accepts the input of body information (height, age, sex) from the subject (step S302). The control section 12 then reads out the weight immediately before from the memory 14 (step S304). The trouble of inputting the weight value by the subject can then be omitted. The data of the weight to be read out may be data immediately before the same time zone or may be simply the data immediately before (irrespective of time zone).

The two limbs impedance measuring unit 102 then measures the impedance between both hands Zh (step S306). The control section 12 then reads out the correction value ZR_h or the correlated information immediately before (recent) of the same time zone from the memory 14 (step S308). The first body composition calculating process is then executed (step S310). The specific process of the first body composition calculating process of step S310 will be described using FIG. 13.

FIG. 13 is a flowchart showing the first body composition calculating process in the first embodiment of the present invention.

With reference to FIG. 13, the correcting unit 104 corrects the impedance between both hands Zh measured in step S306 (step S402). Specifically, the correction value Zr_h read out as correlated information in step S308 is added to the impedance between both hands Zh to calculate the corrected impedance Zh'.

The second body composition calculating unit 105 calculates the body composition of the whole body, that is, the body fat percentage (%FAT_h) based on the corrected impedance Zh' (step S404). More specifically, the body fat percentage is calculated based on the impedance Zh', the body information of the subject, and the equations (1) and (3).

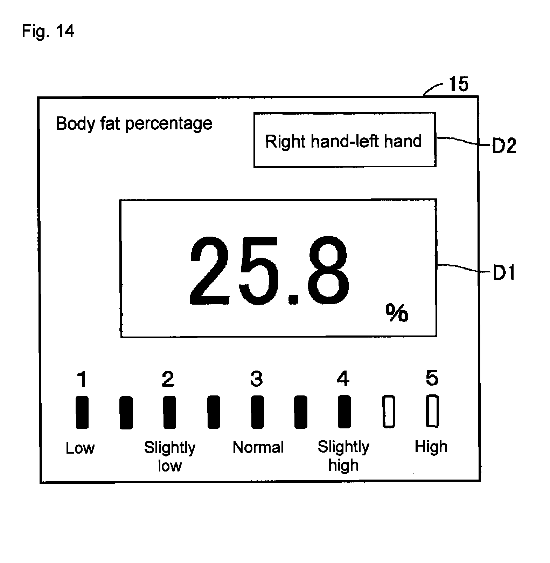

Again referring to FIG. 12, after the first body composition calculating process is terminated, the control section 12 writes the measurement result etc. to the memory 14 in correspondence to the time zone determined in step S4 (step S312). Finally, the informing unit 108 displays the measurement result (body fat percentage) and the information related to the measurement site on the display section 15 (step S314). One example of a display screen in step S314 is shown in FIG. 14.

With reference to FIG. 14, the body fat percentage of the whole body calculated in step S404 is displayed in the display region D1, and characters "right hand-left hand" etc. are displayed as information related to the measurement site in the display region D2 in the display section 15. The subject is then able to recognize the body fat percentage as being calculated based on the detection result of the potential difference between both hands (right hand-left hand). The information of the measurement site itself is displayed as the information related to the measurement site, but information representing the mode such as "hand-simple measurement mode" may be displayed. In this case as well, the subject is able to similarly recognize the body fat percentage as being calculated based on the detection result of the potential difference between both hands. Similar to the display example shown in FIG. 9, the advice information on the evaluation of the calculation result of the body fat percentage may be displayed.

The hand measurement process is then terminated.

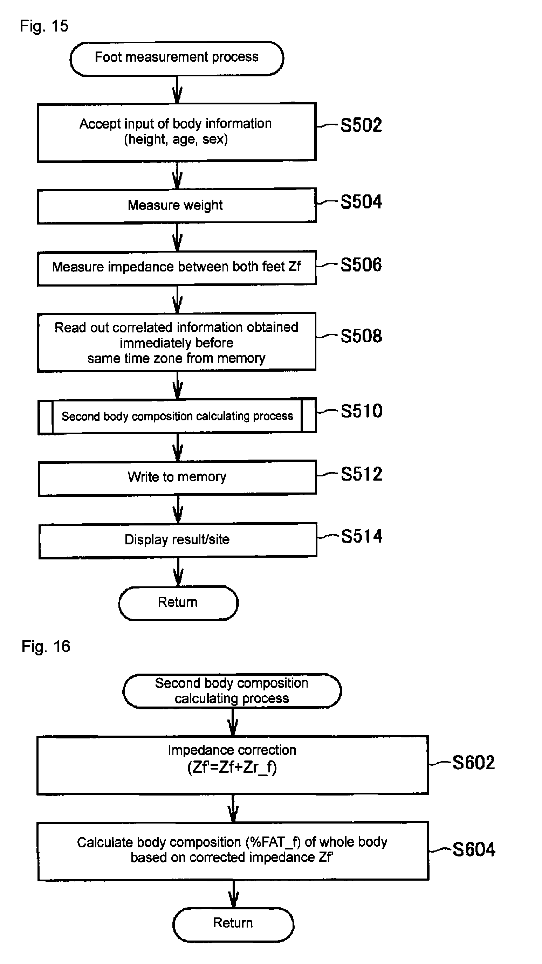

FIG. 15 is a flowchart showing a foot measurement process in the first embodiment of the present invention.

With reference to FIG. 15, the control section 12 accepts the input of body information (height, age, sex) from the subject (step S502). The control section 12 then measures the weigh with the weight measurement section 22 (step S504).

The two limbs impedance measuring unit 102 then measures the impedance between both feet Zf (step S506). The control section 12 then reads out the correction value ZR_f or the correlated information immediately before of the same time zone from the memory 14 (step S508). The second body composition calculating process is then executed (step S510). The specific process of the second body composition calculating process of step S510 will be described using FIG. 16.

FIG. 16 is a flowchart showing the second body composition calculating process in the first embodiment of the present invention.

With reference to FIG. 16, the correcting unit 104 corrects the impedance between both feet Zf measured in step S506 (step S602). Specifically, the correction value Zr_f read out as correlated information in step S508 is added to the impedance between both feet Zh to calculate the corrected impedance Zf'.

The second body composition calculating unit 105 calculates the body composition of the whole body, that is, the body fat percentage (%FAT_f) based on the corrected impedance Zf' (step S604). More specifically, the body fat percentage is calculated based on the impedance Zf', the body information of the subject, and the equations (1) and (4).

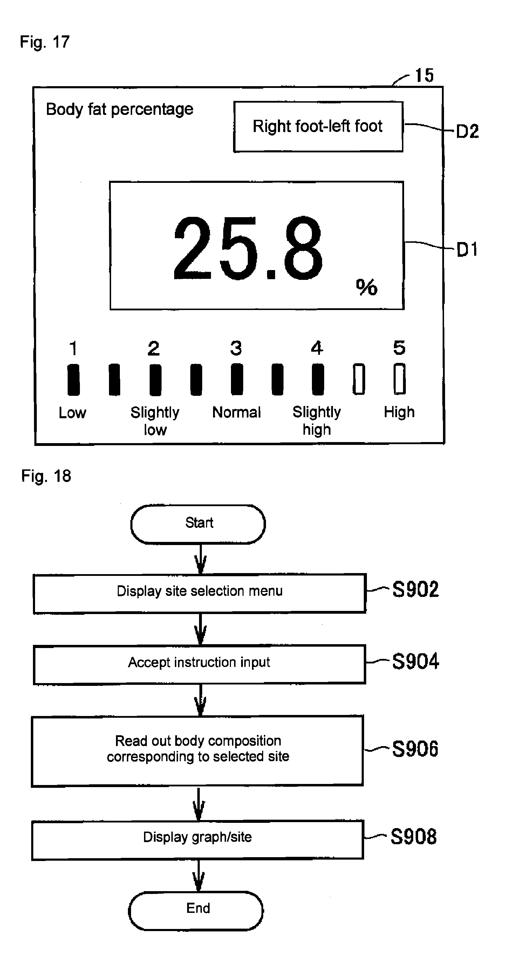

Again referring to FIG. 15, after the second body composition calculating process is terminated, the control section 12 writes the measurement result etc. to the memory 14 in correspondence to the time zone (step S512). Finally, the informing unit 108 displays the measurement result (body fat percentage) and the information related to the measurement site on the display section 15 (step S514). One example of a display screen in step S514 is shown in FIG. 17.

With reference to FIG. 17, the body fat percentage of the whole body calculated in step S604 is displayed in the display region D1, and characters "right foot-left foot" etc. are displayed as information related to the measurement site in the display region D2 in the display section 15. The subject is then able to recognize the body fat percentage as being calculated based on the detection result of the potential difference between both feet (right foot-left foot). The information of the measurement site itself is displayed as the information related to the measurement site, but information representing the mode such as "foot-simple measurement mode" may be displayed. In this case as well, the subject is able to similarly recognize the body fat percentage as being calculated based on the detection result of the potential difference between both feet. Similar to the display example shown in FIG. 9, the advice information on the evaluation of the calculation result of the body fat percentage may be displayed.

The hand measurement process is then terminated.

As described above, in the first embodiment of the present invention, the correction value of the two limbs impedance is set as correlated information in the whole body measurement mode. That is, the correction value of the two limbs impedance such that the body composition of the whole body based on the whole body impedance and the estimated equation (2) matches for the body composition of the whole body based on the two limbs impedance and the estimated equations (3), (4) calculated in the whole body measurement mode is set as the correlated information. The body composition of the whole body of high reliability corresponding to the user (subject) then can be calculated even in the simple measurement mode.

In the present embodiment, characters indicating the measurement site are simultaneously displayed when displaying the calculation result of the body fat percentage on the display section 15. The subject then can easily recognize based on what potential difference of which measurement site the body composition of the whole body is calculated from. The calculation result of the body fat percentage and the information related to the measurement site are displayed on the same screen in the display example described above, but the calculation result of the body fat percentage and the information related to the measurement site may be alternately displayed.

Furthermore, in the present embodiment, influence of daily fluctuation can be absorbed since the correlated information is set for every time zone in measurement. That is, since the correlation value of the two limbs impedance is set as the correlated information such that the body composition of the whole body based on the whole body impedance and the estimated equation (2) and the body composition of the whole body based on the two limbs impedance and the estimated equations (3), (4) match, body composition numerical value of high precision same as the body composition of the whole body calculated based on the whole body impedance and the estimated equation (2) can be estimated even in the simple measurement mode. The subject then can also check change in the body composition of the whole body without being concern of the influence of the daily fluctuation even if the body composition of the whole body is measured in the simple measurement mode.

In the present embodiment, information is made to induce use in the whole body measurement mode when the correlated information of the same time zone set within a predetermined time (e.g., seven days) is not stored in the memory 14 (NO in step S8). However, the informing method is not limited. Information may be made to prohibit use in the simple measurement mode. Alternatively, the body composition of the whole body may be calculated without performing a correction process, and such fact (fact that correction process is not applied) may be informed. More specifically, when referring to a mode name of measuring the body composition of the whole body by using simply the two limbs impedance as "exclusive two limbs measurement mode", information can be made as being the measurement result by the exclusive two limbs measurement mode.

In the present embodiment, presence of the correlated information of the same time zone set within the predetermined time is determined to enhance the reliability, but the presence of the correlated information of the same time zone may be simply determined.

In the present embodiment, the body information is input for every measurement, but the body information that is once input may be stored in the memory 14 so that subsequent inputs can be omitted.

In the present embodiment, the correlated information is set for every time zone, but may be set irrespective of the time zone. Alternatively, the correlated information may be set for every other measurement conditions (before exercise, after exercise, and the like) other than the time zone.

In the present embodiment, the correction value of the two limbs impedance is obtained with the body fat percentage %FAT_w and the body fat percentage %FAT_h, f as the reference when setting the correlated information, but may be obtained with the fat free mass FFM_w and the fat free mass FFM_h, f as the reference. Alternatively, the correction value of the body composition (e.g., fat free mass) of two limbs may be obtained as correlated information. Furthermore, the correction value of the potential difference of the two limbs may be obtained as the correlated information.

In the present embodiment, the correlated information is stored in correspondence to the time zone, and the correlated information corresponding to the time zone determined in the simple measurement mode is read out. However, the correlated information may be stored in correspondence to time, and the correlated information corresponding to the time zone determined in the simple measurement mode may be read out.

In the present embodiment, information related to the measurement site is informed regardless of which body site the measurement site is, but information related to the measurement site may be informed only when the measurement site is the body site other than the whole body (when measurement site is two limbs).

In the present embodiment, both the hand-simple measurement mode and the foot-simple measurement mode are provided for the simple measurement mode, but may be either one. When only the hand-simple measurement mode is provided, determination may be simply made as "whole body measurement mode" if the connector 18 and the connector 31 are connected and as "hand-simple measurement mode" if not connected in the mode determination process described above. Similarly, when only the foot-simple measurement mode is provided, determination may be made as "foot simple measurement mode" if the upper limb unit 1 is accommodated in the accommodating section 20 and as "whole body measurement mode" if not accommodated. Alternatively, a mode of measuring the body composition based on the impedance between right hand-left foot, and the like may be further provided.

(Variant)

A variant of the first embodiment of the present invention will be described below.

In the first embodiment, the value of the calculated body composition and the information related to the measurement site are displayed in association with each other for every measurement, but the value of the body composition measured in the past and the information related to the measurement site may be displayed in association with each other as described below.

FIG. 18 is a flowchart showing a memory readout/display process according to a variant of the first embodiment of the present invention. The process shown in the flowchart of FIG. 18 is stored in advance in the memory 14 as a program, and the function of the memory readout/display process is realized when the control section 12 reads out and executes the relevant program. The processes shown below are contained in the operation section 16, and the like. The process starts in response to the push of a memory switch (not shown) for accepting the display instruction of the past measurement data.

With reference to FIG. 18, the control section 12 displays a site selection menu on the display section 15 (step S902). For instance, buttons respectively representing the whole body, both hands, and both feet are displayed on the display section 15.

The control section 12 then accepts an input of instruction from the user (subject) (input of instruction for selecting one of the whole body, both hands, and both feet) (step S904). The measurement site is selected when the user operates a predetermined switch of the operation section 16.

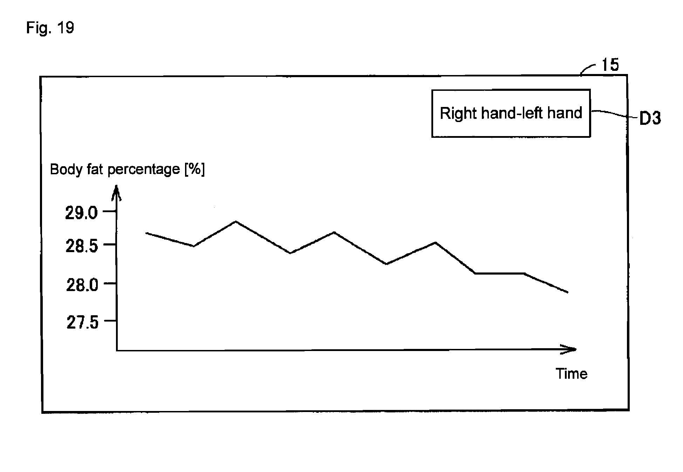

After accepting the instruction, the control section 12 reads out the body composition corresponding to the selected measurement site from the memory 14 (step S906). The informing unit 108 displays the read measurement value in a graph and also displays the information related to the measurement site on the display section 15 (step S908). Specifically, in step S906, the control section 12 reads out the body composition data F associated with the measurement mode data M indicating the selected measurement site over a predetermined number of times. In step S908, the control section 12 plots the read measurement values in association with time (how many times before), so that information on the measurement values according to change with time can be displayed. One example of the display screen in step S908 is shown in FIG. 19.

With reference to FIG. 19, a graph showing the history (transition) of the measurement values having the body fat percentage (unit: %) on the vertical axis and the time on the horizontal axis is displayed on the display section 15. Characters "right hand-left hand" and the like are displayed as information related to the measurement site in a predetermined display region D3. The subject can then recognize that the graph is the history of the body fat percentage calculated based on the detection result of the potential difference at both hands (right hand-left hand), and mix up of the measurement sites (measurement mode) can be prevented. The information of the measurement site itself is displayed as the information related to the measurement site, but information representing the mode such as "hand-simple measurement mode" may be displayed. In this case as well, the subject can similarly recognize the graph as the history of the body fat percentage calculated based on the detection result of the potential difference between both hands.

As described above, description has been made that the process starts when the memory switch (not shown) is pushed in the variant, but a graph showing the history of the past measurement values corresponding to each measurement site may be displayed in a series of body composition measurement processes (e.g., after body composition display). Alternatively, a memory switch may be arranged for every measurement site, and readout and display of the measurement value may be performed according to the selected memory switch.

In the present variant, the history of the past measurement values is displayed in a graph, but the measurement site and the past measurement values merely need to be displayed (informed) in association with each other, and thus is not limited to a graph display. The measurement value corresponding to the site selected in step S904 may be read out one at a time every time that the memory switch (not shown) is pushed, and displayed with the information related to the measurement site.

If the measurement site is the body site other than the whole body, the history of the measurement values in the whole body measurement mode may be further displayed overlapping the history of the measurement values in the simple measurement mode. The extent of difference in the measurement results depending on the difference in the measurement site thus can be known.

Furthermore, the influence of daily fluctuation etc. of the individual subject can be known if the setting of the correlated information in the whole body measurement mode and the correction process in the simple measurement mode as described above are not performed.

[Second embodiment]

A second embodiment of the present invention will now be described.

In the first embodiment, the correlation value of the two limbs impedance is assumed as the correlated information. In the second embodiment the correlation between the body composition of the whole body calculated based on the whole body impedance and the body composition of the whole body calculated based on the two limbs impedance is assumed as the correlated information. The outer appearance and the hardware configuration of the body composition measuring instrument according to the second embodiment are the same as the body composition measuring instrument 100 according to the first embodiment. Therefore, description will be made herein with the reference numerals shown in Figs. 1 and 2.

The difference with the first embodiment will now be described.

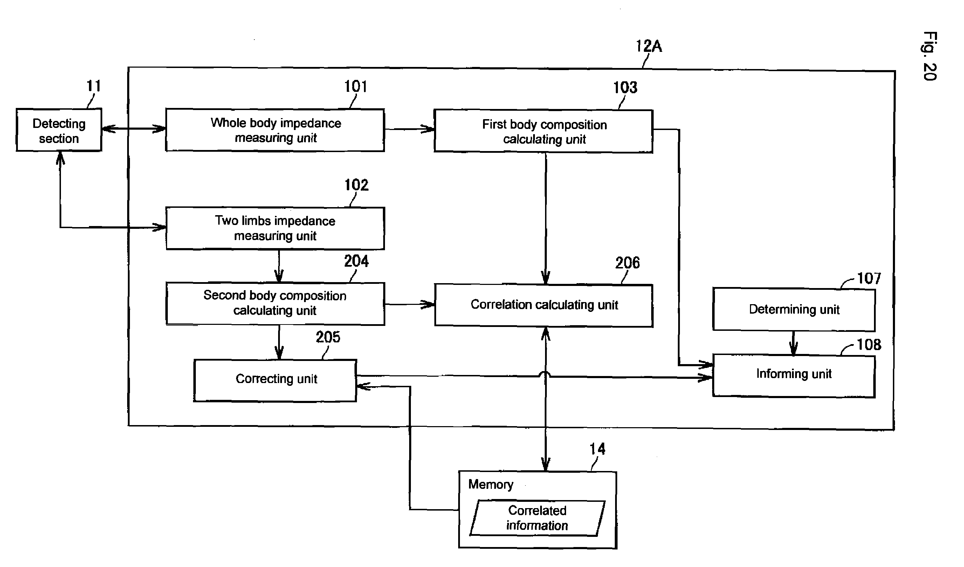

FIG. 20 is a function block diagram of the body composition measuring instrument 100 according to the second embodiment of the present invention. The control section in the second embodiment differs from the function of the control section 12 in the first embodiment. Therefore, it is written as control section 12A in the present embodiment.

With reference to FIG. 20, the control section 12A includes the whole impedance measuring unit 101, the two limbs impedance measuring unit 102, the first body composition calculating unit 103, the determining unit 107, and the informing unit 108, similar to the first embodiment. The control section 12A includes a second body composition calculating unit 204, a correcting unit 205, and a correlation calculating unit 206 in place of the correcting unit 104, the second body composition calculating unit 105, and the correlation setting unit 106 in the first embodiment.

The second body composition calculating unit 204 calculates the body composition of the whole body based on the two limbs impedance measured by the two limbs impedance measuring unit 102.

In the simple measurement mode, the correcting unit 205 corrects the body composition of the whole body calculated in the second body composition calculating unit 204 based on the correlated information stored in the memory 14. (correlation between the body composition of the whole body calculated based on the whole body impedance and the body composition of the whole body calculated based on the two limbs impedance).

The correlation calculating unit 206 calculates the correlation between the body composition of the whole body calculated by the second body composition calculating unit 204 in the whole body measurement mode and the body composition of the whole body calculated by the first body composition calculating unit 103. Specifically, the correlation between the fat free mass FFM_w and the fat free mass FFM_h, f, for example, is calculated by the correlation calculating unit 206. The detailed calculation method will be hereinafter described.

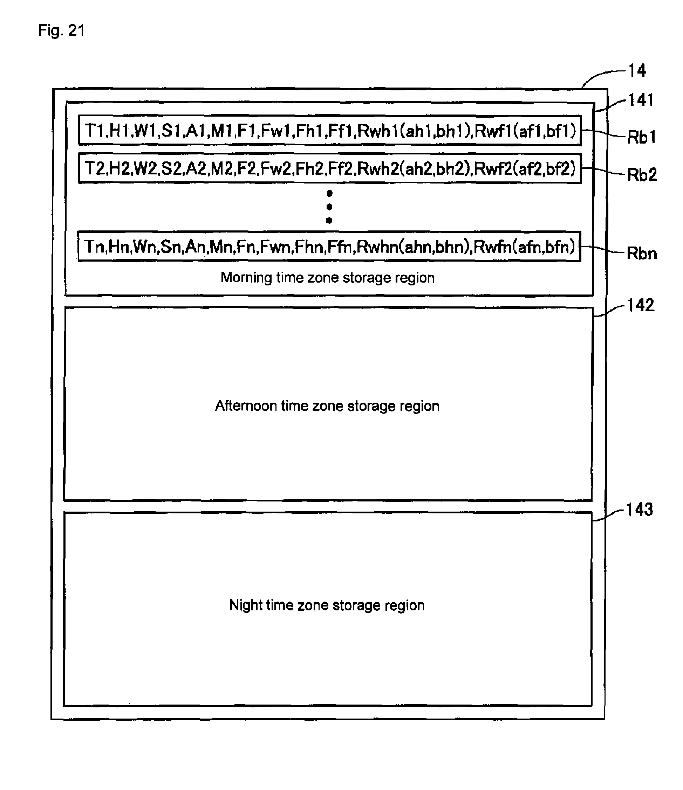

FIG. 21 is a view showing one example of a data structure of the memory 14 in the body composition measuring instrument 100 of the second embodiment of the present invention.

With reference to FIG. 21, the memory 14 includes the morning time zone storage region 141 for storing the measurement result in the morning time zone, the afternoon time zone storage region 142 for storing the measurement result in the afternoon time zone, and the night time zone storage region 143 for storing the measurement result in the night time zone, similar to the first embodiment.

When the body composition measurement process is executed, the records Rb (Rb1, Rb2, ..., Rbn) including date and time data T in measurement, height input value data H serving as body information, weight value data W serving as body information, sex data S serving as body information, age data A serving as body information, measurement mode data M, body composition data F of the whole body serving as measurement result, fat free mass data Fw, Fh, Ff of the whole body, correlated information Rwh, and correlated information Rwf are stored in the region corresponding to the time zone in measurement.

Similar to the first embodiment, the body composition data F of the whole body is the measurement result of the final body composition, and is the data of the body fat percentage calculated by the first body composition calculating unit 103 or the data of the body fat percentage after corrected by the correcting unit 205. That is, it is the calculation result data by the first body composition calculating unit 103 if the measurement mode data is "0" (whole body measurement mode), and is the calculation result data by the correcting unit 205 if the measurement mode data M is "1" or "2" (simple measurement mode).

The fat free mass data Fw of the whole body is the data of the fat free mass FFM_w calculated based on the whole body impedance Zw and the estimated equation (2) by the first body composition calculating unit 103 when the measurement mode data M is "0" (whole body measurement mode). The fat free mass FFM_w is calculated in the calculation of the body fat percentage in step S108. The fat free mass data Fh of the whole body is the data of the fat free mass FFM_h calculated based on the hands impedance Zh and the estimated equation (3) by the second body composition calculating unit 204 when the measurement mode data M is "0" (whole body measurement mode). The fat free mass data Ff of the whole body is the data of the fat free mass FFM calculated based on the impedance between both feet Zf and the estimated equation (4) by the second body composition calculating unit 204 when the measurement mode data M is "0" (whole body measurement mode).

In the second embodiment, the correlated information Rwh and the correlated information Rwf respectively stores the data indicating correlation coefficients ah, bh, and correlation coefficients af, bf, to be hereinafter described.

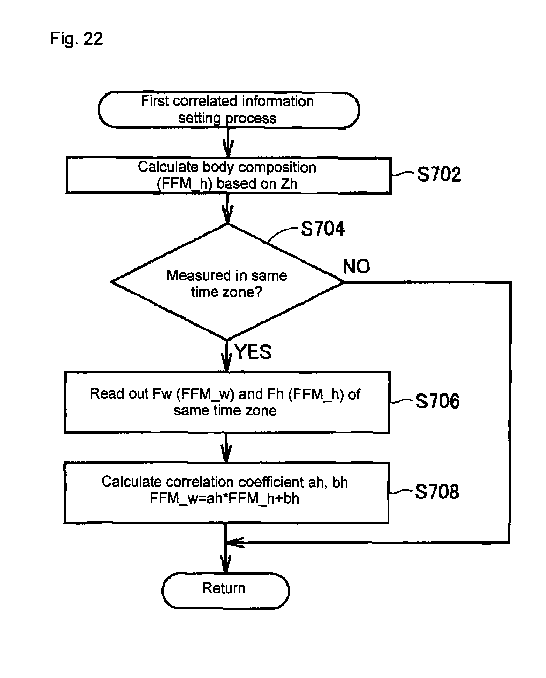

FIG. 22 is a flowchart showing a first correlated information setting process in the second embodiment of the present invention.

With reference to FIG. 22, the second body composition calculating unit 204 calculates the fat free mass (FFM_h) of the whole body based on the impedance between both hands Zh (step S702). The control section 12A determines whether or not measurement in the whole body measurement mode is completed in the same time zone (S704). More specifically, determination is made on whether or not the record Rb in which the mode data M is "0" exists of the records Rb stored in the same time zone. The process proceeds to S706 if determined as measured (YES in S704). On the other hand, the process is terminated if determined as not measured (NO in step S704).

In step S706, the correlation calculating unit 206 reads out all the data Fw of the fat free mass (FFM w) of the whole body and the data Fh of the fat free mass (FFM_h) of the whole body in the same time zone from the memory 14.

Thereafter, the correlation calculating unit 206 calculates the correlation between the fat free mass FFM_w of the whole body and the fat free mass FFM_h of the whole body (step S708). More specifically, the correlation coefficients ah, bh that satisfy the following correlating equation are calculated based on the fat free mass calculated in step S108 and S702, and the fat free mass read out in step S706.

$$FFM\_w = ah*FFM\_h + bh$$

The first correlated information setting process is then terminated.

The calculation of the correlation coefficient can be realized by using a least square method etc. from each data.

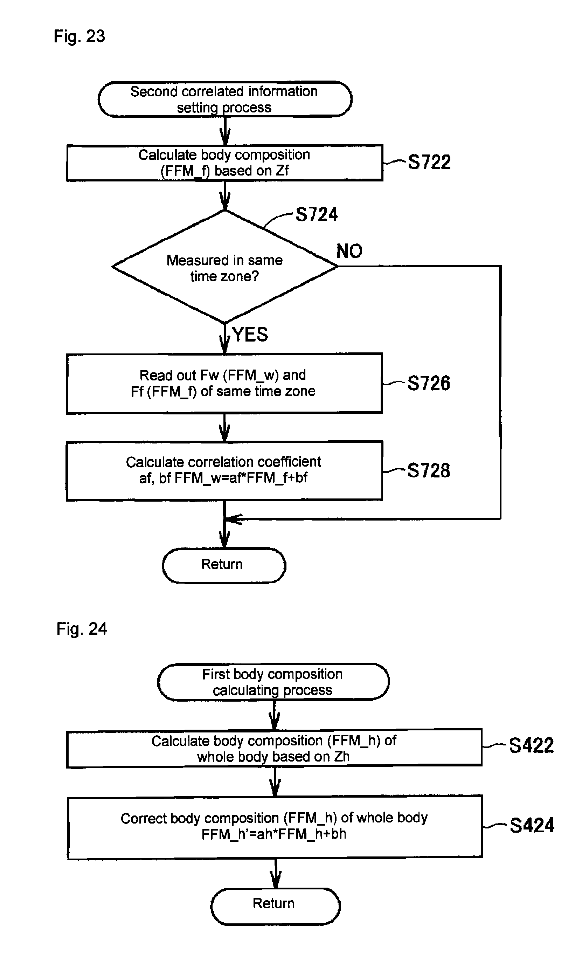

FIG. 23 is a flowchart showing a second correlated information setting process in the second embodiment of the present invention.

With reference to FIG. 23, the second body composition calculating unit 204 calculates the fat free mass (FFM_f) of the whole body based on the impedance between both feet Zf (step S722). The control section 12A determines whether or not measurement in the whole body measurement mode is completed in the same time zone (S724). The process proceeds to S726 if determined as measured (YES in S724). On the other hand, the process is terminated if determined as not measured (NO in step S724).

In step S726, the correlation calculating unit 206 reads out all the data Fw of the fat free mass (FFM_w) of the whole body and the data Ff of the fat free mass (FFM_f) of the whole body in the same time zone.

Thereafter, the correlation calculating unit 206 calculates the correlation between the fat free mass FFM_w of the whole body and the fat free mass FFM_f of the whole body (step S728). More specifically, the correlation coefficients af, bf that satisfy the following correlating equation are calculated based on the fat free mass calculated in step S108 and S722, and the fat free mass read out in step S726.

$$FFM\_w=af*FFM\_f+bf$$

The respective processes of steps S722 to S728 correspond to the processes of steps S702 to S708 shown in FIG. 22. Therefore, detailed description thereof will not be repeated herein.

In the second embodiment, the correlation coefficients ah, bh are stored as the correlated information Rwh, and the correlation coefficients af, bf are stored as the correlated information Rwf in step S118 of FIG. 18 by performing the first and second correlated information setting processes described above. Furthermore, the body fat percentage %FAT_w calculated in step S108 is stored as body fat percentage data F. The fat free mass FFM_w, FFM_h, FFM_f calculated in steps S108, S702, S722 are respectively stored as fat free mass data Fw, Fh, Ff.

FIG. 24 is a flowchart showing a first body composition calculating process in the second embodiment of the present invention. In the second embodiment, the correlation coefficients ah, bh immediately before the same time zone are read out in step S308.

With reference to FIG. 24, the second body composition calculating unit 204 calculates the fat free mass FFM_h of the whole body based on the impedance between both hands Zh measured in step S306 (step S422). More specifically, the fat free mass is calculated based on the impedance between both hands Zh, the body information of the subject, and the equation (3).

The correcting unit 205 corrects the fat free mass FFM_h of the whole body calculated in step S422 based on the correlation coefficients ah, bh read out as correlated information in step S308 (step S424). More specifically, the fat free mass FFM_h' of the whole body after correction is calculated using the following equation.

$$FFM\_h´=ah*FFM\_h+bh$$

In step S424, the body fat percentage %FAT_h is calculated by substituting the corrected fat free mass FFM_h' to equation (1).

When the above processes are executed, the body fat percentage %FAT_h calculated in step S424 is stored in the memory 14 as body composition data F of the whole body in step S312, and presented to the subject in step S314.

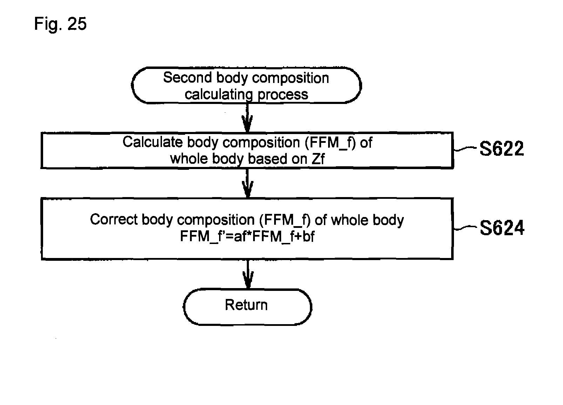

FIG. 25 is a flowchart showing a second body composition calculating process in the second embodiment of the present invention. In the second embodiment, the correlation coefficients af, bf immediately before the same time zone are read out in step S508.

With reference to FIG. 25, the second body composition calculating unit 204 calculates the fat free mass FFM_f of the whole body based on the impedance between both feet Zf measured in step S506 (step S622). More specifically, the fat free mass is calculated based on the impedance between both feet Zf, the body information of the subject, and the equation (4).

The correcting unit 205 corrects the fat free mass FFM_f of the whole body calculated in step S622 based on the correlation coefficients af, bf read out as correlated information in step S508 (step S624). More specifically, the fat free mass FFM_f' of the whole body after correction is calculated using the following equation.

$$FFM\_f'=af*FFM\_f+bf$$

In step S624, the body fat percentage %FAT_f is calculated by substituting the corrected fat free mass FFM_f' to equation (1).

When the above processes are executed, the body fat percentage %FAT_f calculated in step S624 is stored in the memory 14 as body composition data F of the whole body in step S512, and presented to the subject in step S514.

As described above, in the second embodiment of the present invention, the correlation between the body composition of the whole body based on the whole body impedance and the body composition of the whole body based on the two limbs impedance is set as the correlated information in the whole body measurement mode. The body composition of the whole body of high reliability corresponding to the subject then can be calculated even in the simple measurement mode.

In the present embodiment, description has been made in calculating the correlation between the fat free mass FFM_w and the fat free mass FFM_h or FFM_f, but the correlation between the body fat percentage %FAT_w and the body fat percentage %FAT_h or %FAT_f may be calculated.

Furthermore, in the present embodiment, all the data Fw of the fat free mass and the data Fh, Ff of the fat free mass stored in the storage region of the same time zone are read out in steps S706 and S726, but data within a predetermined period in the past may be read out. Alternatively, all the fat free mass data Fw, Fh, Ff within a predetermined period may be further included in the record Rb. Therefore, the data immediately before the same time zone only needs be read out.

[Third embodiment]

A third embodiment of the present invention will now be described.

In the first embodiment, the correlation value of the two limbs impedance is assumed as the correlated information. In the second embodiment, the correlation between the body composition of the whole body calculated based on the whole body impedance and the body composition of the whole body calculated based on the two limbs impedance is assumed as the correlated information.

In the third embodiment, the correlation between the whole impedance and the two limbs impedance is assumed as the correlated information. The outer appearance and the hardware configuration of the body composition measuring instrument according to the third embodiment are the same as the body composition measuring instrument 100 according to the first and the second embodiments. Therefore, description will be made herein with the reference numerals shown in Figs. 1 and 2.

The difference with the first embodiment will be mainly described below.

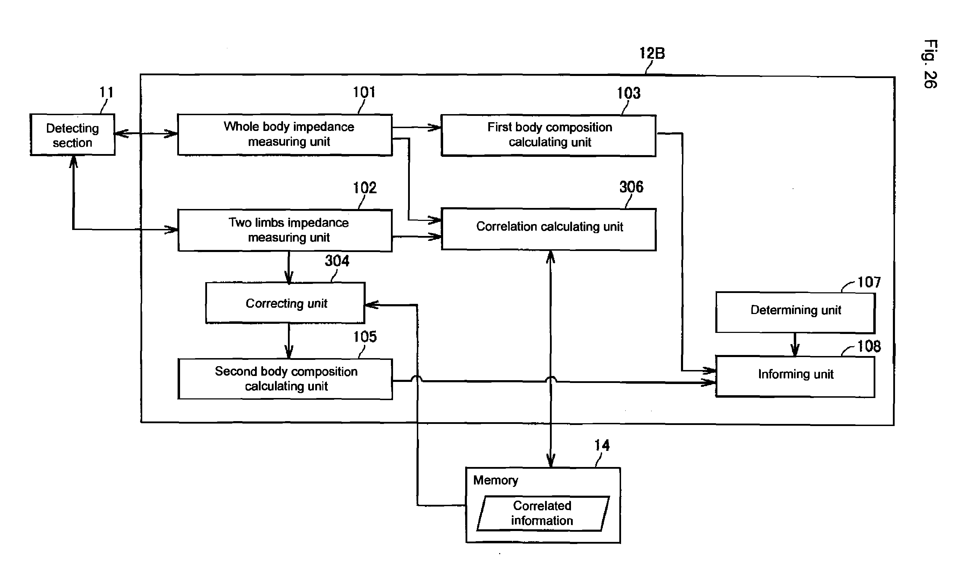

FIG. 26 is a function block diagram of the body composition measuring instrument 100 according to the third embodiment of the present invention. The control section in the third embodiment differs from the function of the control section 12 in the first embodiment and the control section 12A in the second embodiment. Therefore, it is written as control section 12B in the present embodiment.

With reference to FIG. 26, the control section 12B includes the whole impedance measuring unit 101, the two limbs impedance measuring unit 102, the first body composition calculating unit 103, the second body composition calculating unit 105, the determining unit 107, and the informing unit 108, similar to the first embodiment. The control section 12B includes a correcting unit 304 and a correlation calculating unit 306 in place of the correcting unit 104 and the correlation setting unit 106 in the first embodiment.

The correcting unit 304 corrects the two limbs impedance measured by the two limbs impedance measuring unit 102 based on the correlated information (correlation between whole body impedance and two limbs impedance) stored in the memory 14 in the simple measurement mode.

The correlation calculating unit 306 calculates the correlation between the whole body impedance measured by the whole body impedance measuring unit 101 and the two limbs impedance measured by the two limbs impedance measuring unit 102 in the whole body measurement mode.

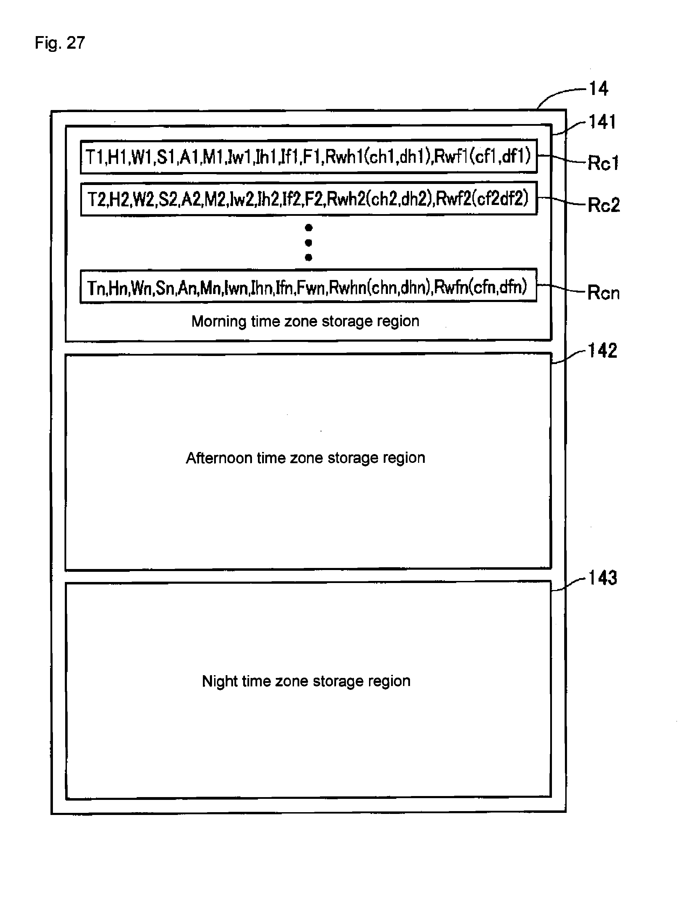

FIG. 27 is a view showing one example of a data structure of the memory 14 in the body composition measuring instrument 100 of the third embodiment of the present invention.

With reference to FIG. 27, the memory 14 includes the morning time zone storage region 141 for storing the measurement result in the morning time zone, the afternoon time zone storage region 142 for storing the measurement result in the afternoon time zone, and the night time zone storage region 143 for storing the measurement result in the night time zone, similar to the first embodiment.

When the body composition measurement process is executed, the records Rc (Rc1, Rc2, ..., Rcn) including date and time data T in measurement, height input value data H serving as body information, weight value data W serving as body information, sex data S serving as body information, age data A serving as body information, measurement mode data M, data Iw indicating the whole body impedance Zw, data Ih indicating the impedance between both hands Zh, data If indicating the impedance between both feet Zf, body composition data F of the whole body serving as measurement result, correlated information Rwh, and correlated information Rwf are stored in the region corresponding to the time zone in measurement.

Similar to the first embodiment, the body composition data F of the whole body is the measurement result of the final body composition, and is the data of the body fat percentage calculated by the first body composition calculating unit 103 or the second body composition calculating unit 105.

The data Iw is the data indicating the whole body impedance Zw measured by the whole body impedance measuring unit 101 when the measurement mode data M is "0" (whole body measurement mode). The data Ih is the data indicating the impedance between both hnads Zh measured by the two limbs impedance measuring unit 102 when the measurement mode data M is "0" (whole body measurement mode). The data If is the data indicating the impedance between both feet Zf measured by the two limbs impedance measuring unit 102 when the measurement mode data M is "0" (whole body measurement mode).

In the third embodiment, the correlated information Rwh and the correlated information Rwf store the data indicating correlation coefficients ch, dh, and correlation coefficients cf, df, to be hereinafter described, respectively.

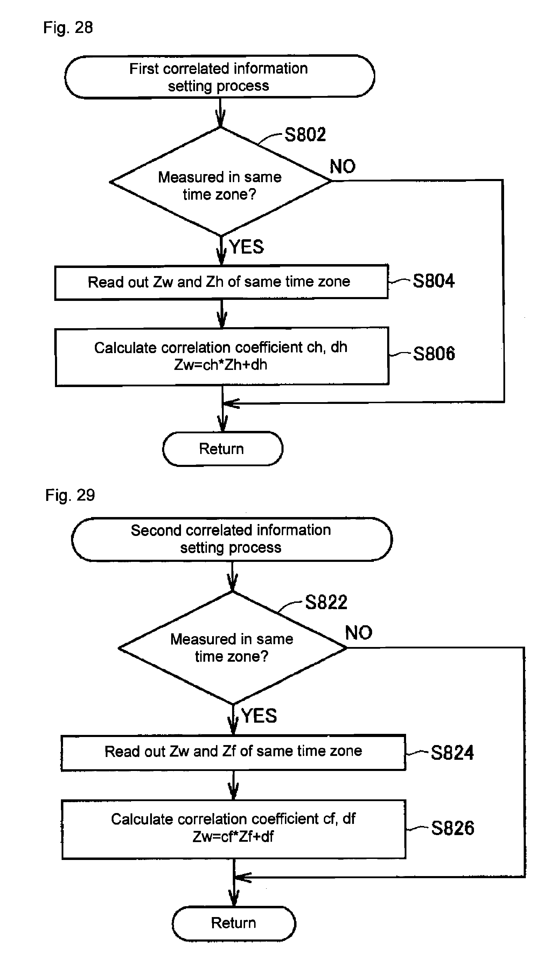

FIG. 28 is a flowchart showing a first correlated information setting process in the third embodiment of the present invention.

With reference to FIG. 28, the control section 12B first determines whether or not measurement in the whole body measurement mode is completed in the same time zone (S802). More specifically, determination is made on whether or not the record Rc in which the mode data M indicates the whole body measurement mode exists of the records Rc stored in the same time zone. The process proceeds to S804 if determined as measured (YES in S802). On the other hand, the process is terminated if determined as not measured (NO in step S802).

In step S804, the correlation calculating unit 306 reads out all the data Iw of the whole body impedance Zw and the data Ih of the impedance between both hands Zh in the same time zone from the memory 14.

Thereafter, the correlation calculating unit 206 calculates the correlation coefficients ch, dh that satisfy the following correlating equation based on the whole body impedance Zw and the impedance between both hands Zh respectively measured in steps S106 and S110, and the whole body impedance Zw and the impedance Zh read out in step S804 (S806).

$$Zw = ch * Zh + dh$$

The first correlated information setting process is then terminated.

FIG. 29 is a flowchart showing a second correlated information setting process in the third embodiment of the present invention.

With reference to FIG. 29, the control section 12B first determines whether or not measurement in the whole body measurement mode is completed in the same time zone (S822). More specifically, determination is made on whether or not the record Rc in which the mode data M indicates the whole body measurement mode exists of the records Rc stored in the same time zone. The process proceeds to S824 if determined as measured (YES in S822). On the other hand, the process is terminated if determined as not measured (NO in step S822).

In step S824, the correlation calculating unit 206 reads out all the data Iw of the whole body impedance Zw and the data If of the impedance between both feet Zf in the same time zone from the memory 14.

Thereafter, the correlation calculating unit 206 calculates the correlation coefficients cf, df that satisfy the following correlating equation based on the whole body impedance Zw and the impedance between both feet Zf respectively measured in steps S106 and S110, and the whole body impedance Zw and the impedance Zf read out in step S824 (S826).

$$Zw = cf * Zf + df$$

The second correlated information setting process is then terminated.

In the third embodiment, the correlation coefficients ch, dh are stored as the correlated information Rwh, and the correlation coefficients cf, df are stored as the correlated information Rwf in step S118 by performing the first and the second correlated information setting processes. Furthermore, the body fat percentage %FAT_W calculated in step S108 is stored as body fat percentage data F. The impedances Zw, Zh, Zf calculated in steps S106, S110, S112 are stored as the data Iw, Ih, If, respectively.

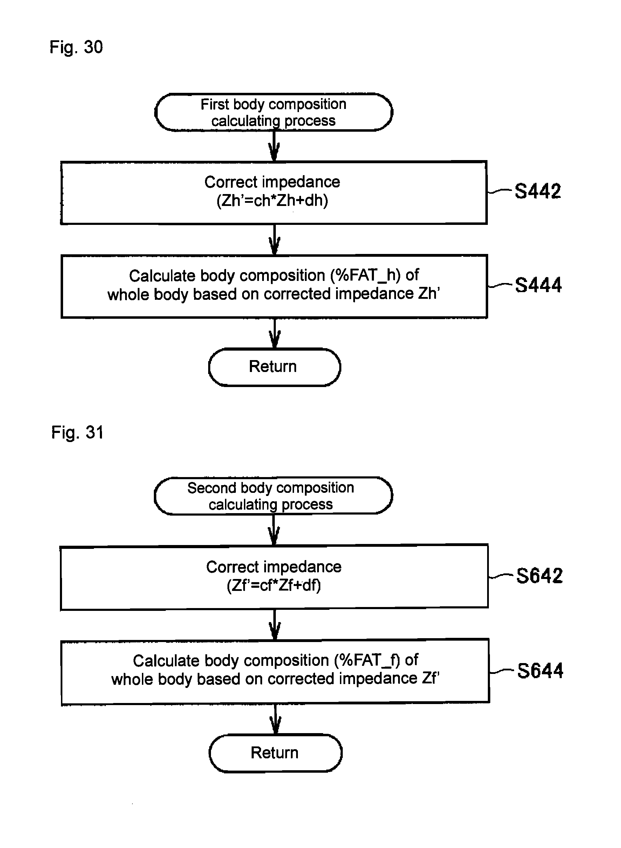

FIG. 30 is a flowchart showing a first body composition calculating process in the third embodiment of the present invention. In the third embodiment, the correlation coefficients ch, dh immediately before the same time zone are read out in step S308.

With reference to FIG. 30, the correcting unit 304 corrects the impedance between both hands Zh measured in step S306 based on the correlation coefficients ch, dh read out as correlated information in step S308 (step S442). More specifically, the impedance Zh' after correction is calculated using the following equation.

$$Zh' = ch \cdot Zh + dh$$

The second body composition calculating unit 105 calculates the body composition (%FAT_h) of the whole body based on the corrected impedance Zh' (step S444). More specifically, the body fat percentage is calculated based on the corrected impedance Zh', the body information of the subject, and the equations (1) and (2) (substitute value of "Zh'" to "Zw" of the estimated equation (2)).

When the above processes are executed, the body fat percentage %FAT_h calculated in step S444 is stored in the memory 14 as body composition data F of the whole body in step S312, and presented to the subject in step S314.

FIG. 31 is a flowchart showing a second body composition calculating process in the third embodiment of the present invention. In the third embodiment, the correlation coefficients cf, df immediately before the same time zone are read out in step S508.

With reference to FIG. 31, the correcting unit 304 corrects the impedance between both feet Zf measured in step S506 based on the correlation coefficients cf, df read out as correlated information in step S508 (step S642). More specifically, the corrected impedance Zf' is calculated using the following equation.

$$Zf' = cf \cdot Zh + df$$

The second body composition calculating unit 105 calculates the body composition (%FAT_f) of the whole body based on the corrected impedance Zf' (step S444). More specifically, the body fat percentage is calculated based on the corrected impedance Zf', the body information of the subject, and the equations (1) and (2) (substitute value of "Zf'" to "Zw" of the estimated equation (2)).

When the above processes are executed, the body fat percentage %FAT_f calculated in step S644 is stored in the memory 14 as body composition data F of the whole body in step S512, and presented to the subject in step S514.

As described above, in the third embodiment of the present invention, the correlation between the whole body impedance and the two limbs impedance is set as the correlated information in the whole body measurement mode. The body composition of the whole body of high reliability corresponding to the subject then can be calculated even in the simple measurement mode.

In the present embodiment, all the data Iw of the whole impedance the data Ih, If of the two limbs impedance stored in the storage region of the same time zone are read out in steps S804 and S824, but data within a predetermined period in the past may be read out. Alternatively, all the impedance data Iw, Ih, If within a predetermined period may be further included in the record Rb. Therefore, the data immediately before the same time zone only needs be read out.

Moreover, in the present embodiment, the correlation between the whole body impedance and the two limbs impedance is set as the correlated information, but the correlation between the potential difference of the whole body and the potential difference of the two limbs may be set as the correlated information.

The body composition measuring instrument 100 of the first to the third embodiments described above have been described such that the body fat percentage is calculated as the body composition of the whole body, but in place thereof or in addition thereto, other biological information such as muscle percentage may be calculated.

The body composition measuring method performed by the composition measuring instrument of the present invention may be provided as a program. Such program may be recorded on an optical medium such as CD-ROM (Compact Disc-ROM) etc., or a computer readable recording medium such as memory card to be provided as a program product. The program may be provided by downloading via a network.

The provided program product is installed in a program storage unit such as a hard disc and then executed.

**EP 1 977 691 A1**

The program product includes the program itself and the recording medium on which the program is recorded.

The embodiments disclosed herein are merely illustrative in all points and should not be construed as restrictive. The scope of the invention is defined by the appended claims rather than the above description, and all changes that fall within meanings and bounds of the claims, or equivalence of such meanings and bounds are therefore intended to be embraced by the claims.

**Claims**

1. A body composition measuring instrument comprising:

   a plurality of hand electrodes (E11 to E14) and a plurality of foot electrodes (E21 to E24);
   a detecting section (11) for detecting a plurality of potential differences at each of a plurality of body sites including a whole body, both hands, and both feet of a subject by using the hand electrodes and the foot electrodes;
   a body composition calculating unit (103, 105) for calculating a body composition of the whole body of the subject based on at least one of the potential differences detected by the detecting section and body information of the subject; and
   an informing unit (108) for informing information related to the body site to be detected of the potential difference used in the calculation of the body composition of the whole body.

2. The body composition measuring instrument according to claim 1, further comprising a determining unit (107) for determining the body site to be detected based on an impedance corresponding to each potential difference and a reference range defined in advance for each body site.

3. The body composition measuring instrument according to claim 1, further comprising:

   a first unit (1) which is arranged with the hand electrodes, the detecting section, and the body composition calculating unit, and which can be gripped by the subject with both hands;
   a second unit (2) which is arranged with the foot electrodes and on which both feet of the subject can be placed;
   a cable (3) for electrically connecting the first unit and the second unit, the cable being removable with respect to the first unit or the second unit;
   a connection detecting unit (19, 107) for detecting presence of connection between the cable and the first unit or the second unit; and
   a determining unit (107) for determining the body site to be detected based on the detection result of the connection detecting unit;

   wherein the determining unit determines the body site to be detected as the whole body when detected as connected by the connection detecting unit, and determines the body site to be detected as both hands when detected as not connected by the connection detecting unit.

4. The body composition measuring instrument according to claim 1, further comprising
   a first unit (1) which is arranged with the hand electrodes and which can be gripped by the subject with both hands;
   a second unit (2) which is arranged with the foot electrodes and on which both feet of the subject can be placed;
   the second unit including,
   an accommodating section (20) for accommodating the first unit, and
   an accommodation detecting unit (21, 107) for detecting whether or not the first unit is accommodated in the accommodating section;
   a cable (3) for electrically connecting the first unit and the second unit; and
   a determining unit (107) for determining the body site to be detected based on the detection result of the accommodation detecting unit;
   wherein the determining unit determines the body site to be detected as both feet when detected as accommodated by the accommodation detecting unit, and determines the body site to be detected as the whole body when detected as not accommodated by the accommodation detecting unit.

5. The body composition measuring instrument according to claim 1,
   wherein the body composition calculating unit includes,
   a first calculating unit (103) for calculating a first body composition of the whole body by using a whole body impedance based on the first potential difference in the whole body,
a correcting unit (104) for correcting a two limbs impedance based on a second potential difference at the body site other than the whole body, and
a second calculating unit (105) for calculating a second body composition of the whole body by using the two limbs impedance corrected by the correcting unit.

6. The body composition measuring instrument according to claim 5,
   wherein
   the first calculating unit calculates the first body composition of the whole body of the subject based on the whole body impedance, the body information of the subject, and a predetermined first estimated equation showing a relationship between the whole body impedance, the body information, and the body composition of the whole body; and
   the body composition measuring instrument further includes,
   a third calculating unit (1061) for calculating a third body composition of the whole body of the subject based on the two limbs impedance based on the second potential difference detected when detecting the first potential difference, the body information of the subject, and a predetermined second estimated equation showing a relationship between the two limbs impedance, the body information, and the body composition of the whole body;
   a correction value calculating unit (1062) for calculating a correction value of the two limbs impedance such that the first body composition of the whole body matches for the third body composition of the whole body; and
   a storage unit (14) for storing the data of the correction value as correlated information.

7. The body composition measuring instrument according to claim 6,
   wherein
   the correcting unit corrects the two limbs impedance based on the data of the correction value; and
   the second calculating unit calculates the second body composition of the whole body of the subject based on the corrected two limbs impedance, the body information of the subject, and the second estimated equation.

8. The body composition measuring instrument according to claim 5,
   wherein
   the first calculating unit calculates the first body composition of the whole body of the subject based on the whole body impedance, the body information of the subject, and a predetermined estimated equation showing a relationship between the whole body impedance, the body information, and the body composition of the whole body; and
   the body composition measuring instrument further includes,
   a correlation calculating unit (106) for calculating a correlation between the whole body impedance and the two limbs impedance based on the second potential difference detected when detecting the first potential difference; and
   a storage unit (14) for storing correlation data representing the correlation as correlated information.

9. The body composition measuring instrument according to claim 8,
   wherein
   the correcting unit corrects the two limbs impedance based on the correlation data; and
   the second calculating unit calculates the second body composition of the whole body based on the corrected two limbs impedance, the body information of the subject, and the estimated equation.

10. The body composition measuring instrument according to claim 1,
    wherein the body composition calculating unit includes,
    a first calculating unit (103) for calculating a first body composition of the whole body by using a whole body impedance based on a first potential difference in the whole body;
    a second calculating unit (105) for calculating a second body composition of the whole body by using a two limbs impedance based on a second potential difference at the body site other than the whole body; and
    a correcting unit (205) for correcting the calculated second body composition of the whole body based on correlation representing a relationship between the first body composition of the whole body and the second body composition of the whole body.

11. The body composition measuring instrument according to claim 10, wherein
    the first calculating unit calculates the first body composition of the whole body of the subject based on the whole body impedance, the body information of the subject, and a predetermined first estimated equation showing a relationship between the whole body impedance, the body information, and the body composition of the whole body;
    the second calculating unit calculates the second body composition of the whole body based on the two limbs impedance, the body information of the subject, and a predetermined second estimated equation showing a relationship between the two limbs impedance, the body information, and the body composition of the whole body; and the body composition measuring instrument further includes,

a correlation calculating unit (206) for calculating a correlation between the first body composition of the whole body and the second body composition of the whole body based on the second potential difference detected when detecting the first potential difference; and

a storage unit for storing correlation data representing the correlation as correlated information.

12. The body composition measuring instrument according to claim 1, further comprising:

a display section (15) for displaying calculation results of the body composition of the whole body;

wherein the informing unit displays information related to the body site to be detected on the display section.

13. The body composition measuring instrument according to claim 1, further comprising:

a voice output section for outputting voice;

wherein the informing unit outputs information related to the body site to be detected to the voice output section by voice.

14. The body composition measuring instrument according to claim 1, further comprising a storage unit (14) for storing the calculated body composition of the whole body and the information related to the body site to be detected in correspondence to each other.

15. The body composition measuring instrument according to claim 14, further comprising:

a readout section (12) for reading out the body composition of the whole body stored in the storage unit;

wherein the informing unit simultaneously informs the read out body composition of the whole body and the information related to the body site to be detected stored in correspondence to the body composition of the whole body.

Fig. 1

100

E11    15    E12

10b

10c

E13    E14

16.1

16

16.2

10a

31

3

21    20

E21

2

E22

E23

E24

Fig. 2

Fig. 3

Fig. 4

14
141

| T1,H1,W1,S1,A1,M1,F1,Rwh1(Zr_h1),Rwf1(Zr_f1) | Ra1 |

| T2,H2,W2,S2,A2,M2,F2,Rwh2(Zr_h2),Rwf2(Zr_f2) | Ra2 |

$\vdots$

| Tn,Hn,Wn,Sn,An,Mn,Fn,Rwhn(Zr_hn),Rwfn(Zr_fn) | Ran |

Morning time zone storage region

142

Afternoon time zone storage region

143

Night time zone storage region

Fig. 5

EP 1 977 691 A1

```
                    ┌──────────┐
                    │  Start   │
                    └──────────┘
                         │
                         ▼
         ┌─────────────────────────────┐
         │  Mode determination process │──S2
         └─────────────────────────────┘
                         │
                         ▼
         ┌─────────────────────────────┐
         │     Determine time zone     │──S4
         └─────────────────────────────┘
                         │        S6
                         ▼
  "Error"         ╱─────────────╲      "Hand-simple", "foot-simple"
         ◄────────┤    Mode?    ├──────────────────────►
                  ╲─────────────╱
                         │ "Whole body"
```

Induce whole body measurement mode — S10

Any correlated information of same time zone set within past seven days? — S8   NO

YES

S12 — Measurement in whole body measurement mode (whole body measurement process)

S14 — Measurement in hand-simple measurement mode (hand measurement process)   "Hand-simple"

S16 — Measurement in foot-simple measurement mode (foot measurement process)   "Foot-simple"

End

Fig. 6

Fig. 7

Fig. 8

```
        ┌─────────────────────────┐
        │  Whole body measurement │
        │       process           │
        └─────────────────────────┘
                    ↓
    ┌───────────────────────────────┐
    │ Accept input of body information │ ─── S102
    │      (height, age, sex)          │
    └───────────────────────────────┘
                    ↓
    ┌───────────────────────────────┐
    │         Measure weight          │ ─── S104
    └───────────────────────────────┘
                    ↓
    ┌───────────────────────────────┐
    │  Measure whole body impedance Zw │ ─── S106
    └───────────────────────────────┘
                    ↓
    ┌───────────────────────────────┐
    │    Calculate body composition    │ ─── S108
    │ (FFM_w, %FAT_w) of whole body based │
    │    on whole body impedance Zw    │
    └───────────────────────────────┘
                    ↓
    ┌───────────────────────────────┐
    │Measure impedance between bothhands Zh│ ─── S110
    └───────────────────────────────┘
                    ↓
    ┌───────────────────────────────┐
    │Measure impedance between both feet Zf│ ─── S112
    └───────────────────────────────┘
                    ↓
    ┌───────────────────────────────┐
    │     First correlated information │ ─── S114
    │         setting process          │
    └───────────────────────────────┘
                    ↓
    ┌───────────────────────────────┐
    │    Second correlated information │ ─── S116
    │         setting process          │
    └───────────────────────────────┘
                    ↓
    ┌───────────────────────────────┐
    │   Write to memory in correspondence │ ─── S118
    │          to memory               │
    └───────────────────────────────┘
                    ↓
    ┌───────────────────────────────┐
    │        Display result/site       │ ─── S120
    └───────────────────────────────┘
                    ↓
            ┌───────────┐
            │   Return   │
            └───────────┘
```

Fig. 9

Fig. 10

First correlated information
setting process

↓

Calculate body composition (%FAT_h) based on Zh ——S202

↓

Read out correction value Zr_h obtained immediately
before same time zone ——S204

↓

——S206

$|\%FAT\_h - \%FAT\_w| > Th\_h$     NO

↓YES                           ——S208

Calculate Zhα such that %FAT_h=%FAT_w

↓                              ——S210              ——S212

Calculate correction value Zr_hα=Zhα-Zh      $Zr\_h\,\alpha = 0$

↓                              ——S214

Update correction value Zr_h

↓

Return

Fig. 11

```
            ╭─────────────────────────────╮
            │   Second correlated information  │
            │        setting process          │
            ╰─────────────────────────────╯
                          │
                          ▼
      ┌──────────────────────────────────────────┐
      │ Calculate body composition (%FAT_f) based on Zf │───S222
      └──────────────────────────────────────────┘
                          │
                          ▼
      ┌──────────────────────────────────────────┐
      │ Read out correction value Zr_f obtained immediately │───S224
      │          before same time zone             │
      └──────────────────────────────────────────┘
                          │
                          ▼
                      S226
            ◇─────────────────────────◇          NO
            │  |%FAT_f−%FAT_w|>Th_f   │─────────────┐
            ◇─────────────────────────◇             │
                          │ YES                      │
                          ▼         S228             │
      ┌──────────────────────────────────────────┐  │
      │   Calculate Zfα such that %FAT_f=%FAT_w   │  │
      └──────────────────────────────────────────┘  │
                          │         S230             │    S232
                          ▼                          ▼
      ┌──────────────────────────────────────┐  ┌──────────────┐
      │  Calculate correction value Zr_fα=Zfα-Zf │  │   Zr_fα =0   │
      └──────────────────────────────────────┘  └──────────────┘
                          │◄─────────────────────────┘
                          ▼         S234
      ┌──────────────────────────────────────────┐
      │          Update correction value Zr_f       │
      └──────────────────────────────────────────┘
                          │
                          ▼
                    ╭──────────╮
                    │  Return  │
                    ╰──────────╯
```

Fig. 12

```
        ( Hand measurement process )
                    │
                    ▼
    ┌─────────────────────────────┐
    │  Accept input of body        │──S302
    │  information (height, age,    │
    │  sex)                         │
    └─────────────────────────────┘
                    │
                    ▼
    ┌─────────────────────────────┐
    │  Read out weight obtained     │──S304
    │  immediately before from      │
    │  memory                       │
    └─────────────────────────────┘
                    │
                    ▼
    ┌─────────────────────────────┐
    │  Measure impedance between    │──S306
    │  both hands Zh                │
    └─────────────────────────────┘
                    │
                    ▼
    ┌─────────────────────────────┐
    │  Read out correlated          │──S308
    │  information obtained         │
    │  immediately before same      │
    │  time zone from memory        │
    └─────────────────────────────┘
                    │
                    ▼
    ┌─────────────────────────────┐
    │ First body composition        │──S310
    │ calculating process           │
    └─────────────────────────────┘
                    │
                    ▼
    ┌─────────────────────────────┐
    │  Write to memory              │──S312
    └─────────────────────────────┘
                    │
                    ▼
    ┌─────────────────────────────┐
    │  Display result/site          │──S314
    └─────────────────────────────┘
                    │
                    ▼
            (  Return  )
```

Fig. 13

```
        ( First body composition )
        (   calculating process  )
                    │
                    ▼
    ┌─────────────────────────────┐
    │  Impedance correction         │──S402
    │  (Zh'=Zh+Zr_h)                │
    └─────────────────────────────┘
                    │
                    ▼
    ┌─────────────────────────────┐
    │  Calculate body composition   │──S404
    │  (%FAT_h) of whole body       │
    │  based on corrected           │
    │  impedance Zh'                │
    └─────────────────────────────┘
                    │
                    ▼
            (  Return  )
```

Fig. 14

Fig. 15

```
        ┌────────────────────────────┐
        │   Foot measurement process │
        └────────────────────────────┘
                      │
                      ▼
    ┌────────────────────────────────┐
    │  Accept input of body information │──── S502
    │        (height, age, sex)        │
    └────────────────────────────────┘
                      │
                      ▼
    ┌────────────────────────────────┐
    │         Measure weight          │──── S504
    └────────────────────────────────┘
                      │
                      ▼
    ┌────────────────────────────────┐
    │ Measure impedance between both feet Zf │──── S506
    └────────────────────────────────┘
                      │
                      ▼
    ┌────────────────────────────────┐
    │ Read out correlated information obtained │
    │          immediately before       │──── S508
    │       same time zone from memory  │
    └────────────────────────────────┘
                      │
                      ▼
    ┌────────────────────────────────┐
    │ Second body composition calculating process │──── S510
    └────────────────────────────────┘
                      │
                      ▼
    ┌────────────────────────────────┐
    │         Write to memory         │──── S512
    └────────────────────────────────┘
                      │
                      ▼
    ┌────────────────────────────────┐
    │        Display result/site      │──── S514
    └────────────────────────────────┘
                      │
                      ▼
              ┌──────────────┐
              │    Return    │
              └──────────────┘
```

Fig. 16

```
        ┌────────────────────────────┐
        │   Second body composition  │
        │     calculating process    │
        └────────────────────────────┘
                      │
                      ▼
    ┌────────────────────────────────┐
    │       Impedance correction      │
    │        (Zf'=Zf+Zr_f)            │──── S602
    └────────────────────────────────┘
                      │
                      ▼
    ┌────────────────────────────────┐
    │ Calculate body composition (%FAT_f) of whole body │──── S604
    │     based on corrected impedance Zf'  │
    └────────────────────────────────┘
                      │
                      ▼
              ┌──────────────┐
              │    Return    │
              └──────────────┘
```

Fig. 17

Body fat percentage

Right foot-left foot — D2

25.8 % — D1

| 1 | 2 | 3 | 4 | 5 |
|---|---|---|---|---|
| Low | Slightly low | Normal | Slightly high | High |

Fig. 18

Start

Display site selection menu — S902

Accept instruction input — S904

Read out body composition corresponding to selected site — S906

Display graph/site — S908

End

Fig. 19

_15

Right hand-left hand ── D3

Body fat percentage [%]

29.0
28.5
28.0
27.5

Time

Fig. 20

Fig. 21

14

141

T1,H1,W1,S1,A1,M1,F1,Fw1,Fh1,Ff1,Rwh1(ah1,bh1),Rwf1(af1,bf1) — Rb1

T2,H2,W2,S2,A2,M2,F2,Fw2,Fh2,Ff2,Rwh2(ah2,bh2),Rwf2(af2,bf2) — Rb2

Tn,Hn,Wn,Sn,An,Mn,Fn,Fwn,Fhn,Ffn,Rwhn(ahn,bhn),Rwfn(afn,bfn) — Rbn

Morning time zone storage region

142

Afternoon time zone storage region

143

Night time zone storage region

Fig. 22

```
        ╭──────────────────────────────╮
        │  First correlated information │
        │       setting process         │
        ╰──────────────────────────────╯
                        │
                        ▼
        ┌──────────────────────────────┐
        │   Calculate body composition  │──── S702
        │    (FFM_h) based on Zh         │
        └──────────────────────────────┘
                        │
                        ▼  ── S704
                  ◇──────────────◇
                 ╱                 ╲          NO
                ╱  Measured in same  ╲──────────────────┐
                ╲    time zone?       ╱                   │
                 ╲                   ╱                    │
                  ◇──────────────◇                        │
                        │ YES                             │
                        ▼                                 │
        ┌──────────────────────────────┐                 │
        │ Read out Fw (FFM_w) and Fh    │──── S706        │
        │ (FFM_h) of same time zone      │                 │
        └──────────────────────────────┘                 │
                        │                                 │
                        ▼                                 │
        ┌──────────────────────────────┐                 │
        │ Calculate correlation         │──── S708        │
        │ coefficient ah, bh             │                 │
        │ FFM_w=ah*FFM_h+bh              │                 │
        └──────────────────────────────┘                 │
                        │◄────────────────────────────────┘
                        ▼
                 ╭──────────────╮
                 │    Return     │
                 ╰──────────────╯
```

Fig. 23

```
        ┌─────────────────────────────┐
        │  Second correlated information │
        │      setting process         │
        └─────────────────────────────┘
                      │
                      ▼
        ┌─────────────────────────────┐
        │   Calculate body composition │──── S722
        │    (FFM_f) based on Zf       │
        └─────────────────────────────┘
                      │
                      ▼            ── S724
                  ◇─────────◇
                 ╱           ╲        NO
                ╱ Measured in  ╲──────────────┐
                ╲ time zone?   ╱              │
                 ╲           ╱                │
                  ◇─────────◇                 │
                      │ YES                   │
                      ▼                       │
        ┌─────────────────────────────┐       │
        │   Read out Fw (FFM_w) and    │──── S726 │
        │  Ff (FFM_f) of same time zone│       │
        └─────────────────────────────┘       │
                      │                        │
                      ▼                        │
        ┌─────────────────────────────┐       │
        │ Calculate correlation coefficient│── S728 │
        │ af, bf FFM_w=af*FFM_f+bf     │       │
        └─────────────────────────────┘       │
                      │◄──────────────────────┘
                      ▼
               ┌───────────┐
               │  Return   │
               └───────────┘
```

Fig. 24

```
        ┌─────────────────────────────┐
        │    First body composition    │
        │     calculating process      │
        └─────────────────────────────┘
                      │
                      ▼
        ┌─────────────────────────────┐
        │ Calculate body composition (FFM_h) of │── S422
        │   whole body based on Zh     │
        └─────────────────────────────┘
                      │
                      ▼
        ┌─────────────────────────────┐
        │ Correct body composition (FFM_h) of whole body │── S424
        │   FFM_h'=ah*FFM_h+bh         │
        └─────────────────────────────┘
                      │
                      ▼
               ┌───────────┐
               │  Return   │
               └───────────┘
```

Fig. 25

```
         ┌─────────────────────────────┐
         │  Second body composition    │
         │  calculating process        │
         └─────────────────────────────┘
                        │
                        ▼
  ┌──────────────────────────────────────────┐
  │  Calculate body composition (FFM_f) of    │──S622
  │  whole body based on Zf                    │
  └──────────────────────────────────────────┘
                        │
                        ▼
  ┌──────────────────────────────────────────┐
  │  Correct body composition (FFM_f) of whole body │──S624
  │  FFM_f'=af*FFM_f+bf                        │
  └──────────────────────────────────────────┘
                        │
                        ▼
              ┌──────────────┐
              │    Return    │
              └──────────────┘
```

Fig. 26

Fig. 27

14
141

T1,H1,W1,S1,A1,M1,Iw1,Ih1,If1,F1,Rwh1(ch1,dh1),Rwf1(cf1,df1) — Rc1

T2,H2,W2,S2,A2,M2,Iw2,Ih2,If2,F2,Rwh2(ch2,dh2),Rwf2(cf2df2) — Rc2

Tn,Hn,Wn,Sn,An,Mn,Iwn,Ihn,Ifn,Fwn,Rwhn(chn,dhn),Rwfn(cfn,dfn) — Rcn

Morning time zone storage region

142

Afternoon time zone storage region

143

Night time zone storage region

Fig. 28

```
        ┌─────────────────────────────┐
        │   First correlated information │
        │        setting process        │
        └─────────────────────────────┘
                      │
                      ▼          ─S802
                   ╱╲
                  ╱    ╲
                 ╱ Measured╲      NO
                ╱  in same   ╲──────────────┐
                ╲ time zone? ╱              │
                 ╲          ╱               │
                  ╲        ╱                │
                   ╲     ╱                  │
                   │ YES                    │
                   ▼                        │
        ┌─────────────────────────────┐     │
        │ Read out Zw and Zh of same   │──S804
        │        time zone             │     │
        └─────────────────────────────┘     │
                      │                      │
                      ▼                      │
        ┌─────────────────────────────┐     │
        │ Calculate correlation        │     │
        │ coefficient ch, dh           │──S806
        │      Zw=ch*Zh+dh             │     │
        └─────────────────────────────┘     │
                      │◄─────────────────────┘
                      ▼
               ┌───────────┐
               │  Return   │
               └───────────┘
```

Fig. 29

```
        ┌─────────────────────────────┐
        │  Second correlated information│
        │        setting process        │
        └─────────────────────────────┘
                      │
                      ▼          ─S822
                   ╱╲
                  ╱    ╲
                 ╱ Measured╲      NO
                ╱  in same   ╲──────────────┐
                ╲ time zone? ╱              │
                 ╲          ╱               │
                  ╲        ╱                │
                   ╲     ╱                  │
                   │ YES                    │
                   ▼                        │
        ┌─────────────────────────────┐     │
        │ Read out Zw and Zf of same   │──S824
        │        time zone             │     │
        └─────────────────────────────┘     │
                      │                      │
                      ▼                      │
        ┌─────────────────────────────┐     │
        │ Calculate correlation        │     │
        │ coefficient cf, df           │──S826
        │      Zw=cf*Zf+df             │     │
        └─────────────────────────────┘     │
                      │◄─────────────────────┘
                      ▼
               ┌───────────┐
               │  Return   │
               └───────────┘
```

Fig. 30

```
┌─────────────────────────────────┐
│      First body composition     │
│       calculating process       │
└─────────────────────────────────┘
                 │
                 ▼
┌─────────────────────────────────┐
│        Correct impedance        │──── S442
│        (Zh'=ch*Zh+dh)           │
└─────────────────────────────────┘
                 │
                 ▼
┌─────────────────────────────────┐
│  Calculate body composition (%FAT_h) of │──── S444
│  whole body based on corrected impedance Zh' │
└─────────────────────────────────┘
                 │
                 ▼
            ┌─────────┐
            │ Return  │
            └─────────┘
```

Fig. 31

```
┌─────────────────────────────────┐
│     Second body composition     │
│       calculating process       │
└─────────────────────────────────┘
                 │
                 ▼
┌─────────────────────────────────┐
│        Correct impedance        │──── S642
│        (Zf'=cf*Zf+df)           │
└─────────────────────────────────┘
                 │
                 ▼
┌─────────────────────────────────┐
│  Calculate body composition (%FAT_f) of │──── S644
│  whole body based on corrected impedance Zf' │
└─────────────────────────────────┘
                 │
                 ▼
            ┌─────────┐
            │ Return  │
            └─────────┘
```

**INTERNATIONAL SEARCH REPORT**

| International application No. |
| --- |
| PCT/JP2007/050240 |

A. CLASSIFICATION OF SUBJECT MATTER
*A61B5/05*(2006.01)i

According to International Patent Classification (IPC) or to both national classification and IPC

B. FIELDS SEARCHED

Minimum documentation searched (classification system followed by classification symbols)
A61B5/05

Documentation searched other than minimum documentation to the extent that such documents are included in the fields searched

| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2005-230120 A (Tanita Corp.), 02 September, 2005 (02.09.05), Full text; all drawings (Family: none) | 1-15 |
| A | JP 2001-178696 A (Tanita Corp.), 03 July, 2001 (03.07.01), Par. Nos. [0008] to [0025]; all drawings & US 6490481 B1 & US 2003/0073925 A1 & EP 1092387 A1 & EP 1466554 A1 & EP 1466555 A1 & DE 60012283 D | 1-15 |
| A | JP 2001-157672 A (Omron Corp.), 12 June, 2001 (12.06.01), Par. Nos. [0018] to [0021]; Figs. 4 to 7 (Family: none) | 1-15 |

☐ Further documents are listed in the continuation of Box C.  ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 25 January, 2007 (25.01.07) | 06 February, 2007 (06.02.07) |

| Name and mailing address of the ISA/<br>Japanese Patent Office | Authorized officer |
| --- | --- |
| Facsimile No. | Telephone No. |

Form PCT/ISA/210 (second sheet) (April 2005)s

**REFERENCES CITED IN THE DESCRIPTION**

**Patent documents cited in the description**

- JP 2005230120 A